# FASCICULE DE BREVET EUROPEEN

(11) **EP 1 233 962 B1**
(45) Date de publication et mention de la délivrance du brevet: **01.03.2006**
(21) Numéro de dépôt: 00974646.2
(22) Date de dépôt: 03.11.2000
(51) Int. Cl.: C07D 407/12, A61K 31/35, A61K 31/40, A61K 31/42, A61K 31/34, C07D 307/24, C07D 405/12, C07D 413/12, C07D 263/24, A61P 39/06

(54) **COMPOSES HETEROCYCLIQUES ET LEUR APPLICATION A TITRE DE MEDICAMENTS**
HETEROCYCLISCHE VERBINDUNGEN UND IHRE VERWENDUNG ALS MEDIKAMENTE
NOVEL HETEROCYCLIC COMPOUNDS AND THEIR USE AS MEDICINES

(30) Priorité: 05.11.1999 FR 9913858; 23.05.2000 FR 0006535
(43) Date de publication de la demande: 28.08.2002
(60) Demande divisionnaire: 05077194.8
(73) Titulaire: SOCIETE DE CONSEILS DE RECHERCHES ET D'APPLICATIONS SCIENTIFIQUES (S.C.R.A.S.), 75016 Paris (FR)
(72) Inventeur: AUVIN, Serge, F-91730 Mauchamps (FR); CHABRIER de LASSAUNIERE, Pierre-Etienne, F-75016 Paris (FR)
(74) Mandataire: Bourgouin, André
(86) Numéro de dépôt international: PCT/FR2000/003067
(87) Numéro de publication internationale: WO 2001/032654

(56) Documents cités:
- EP-A- 0 603 873
- EP-A- 0 641 800
- EP-A- 0 925 786
- WO-A-96/21655
- WO-A-98/25899
- US-A- 5 691 368
- PEET N P ET AL: "Hydroxyoxazolidines as alpha-aminoacetaldehyde equivalents: novel inhibitors of calpain" BIOORGANIC & MEDICINAL CHEMISTRY LETTERS,GB,OXFORD, vol. 9, no. 16, 16 août 1999 (1999-08-16), pages 2365-2370, XP004174192 ISSN: 0960-894X

## Description

La présente invention concerne de nouveaux dérivés hétérocycliques présentant une activité inhibitrice des calpaïnes et/ou une activité piégeuse des formes réactives de l'oxygène (ROS pour "reactive oxygen species "). L'invention concerne également leurs méthodes de préparation, les préparations pharmaceutiques les contenant et leur utilisation à des fins thérapeutiques, en particulier en tant qu'inhibiteurs de calpaïnes et piégeurs de formes réactives de l'oxygène de manière sélective ou non.

Compte tenu du rôle potentiel des calpaïnes et des ROS en physiopathologie, les nouveaux dérivés selon l'invention peuvent produire des effets bénéfiques ou favorables dans le traitement de pathologies où ces enzymes et / ou ces espèces radicalaires sont impliquées, et notamment :
- les maladies inflammatoires et immunologiques comme par exemple l'arthrite rhumatoïde, les pancréatites, la sclérose en plaques, les inflammations du système gastro-intestinal (colite ulcérative ou non, maladie de Crohn),
- les maladies cardio-vasculaires et cérébro-vasculaires comprenant par exemple l'hypertension artérielle, le choc septique, les infarctus cardiaques ou cérébraux d'origine ischémique ou hémorragique, les ischémies ainsi que les troubles liés à l'agrégation plaquettaire,
- les troubles du système nerveux central ou périphérique comme par exemple les maladies neurodégénératives où l'on peut notamment citer les traumatismes cérébraux ou de la moelle épinière, l'hémorragie sub arachnoïde, l'épilepsie, le vieillissement, les démences séniles, y compris la maladie d'Alzheimer, la chorée de Huntington, la maladie de Parkinson, les neuropathies périphériques,
- l'ostéoporose,
- les dystrophies musculaires,
- les maladies prolifératives comme par exemple l'athérosclérose ou la resténose,
- la cataracte,
- les transplantations d'organes,
- les maladies auto-immunes et virales comme par exemple le lupus, le sida, les infections parasitaires et virales, le diabète et ses complications, la sclérose en plaques,
- le cancer,
- toutes les pathologies caractérisées par une production excessive des ROS et / ou une activation des calpaïnes.

Dans l'ensemble de ces pathologies, il existe des évidences expérimentales démontrant l'implication des ROS (Free Radic. Biol. Med. (1996) 20, 675-705 ; Antioxid. Health. Dis. (1997) 4 (Handbook of Synthetic Antioxidants), 1-52) ainsi que l'implication des calpaïnes (Trends Pharmacol. Sci. (1994) 15, 412419 ; Drug News Perspect (1999) 12, 73-82). A titre d'exemple, les lésions cérébrales associées à l'infarctus cérébral ou au traumatisme crânien expérimental sont réduites par des agents antioxydants (Acta. Physiol. Scand. (1994) 152, 349-350 ; J. Cereb. Blood Flow Metabol. (1995) 15, 948-952 ; J Pharmacol Exp Ther (1997) 2, 895-904) ainsi que par des inhibiteurs de calpaïnes (Proc Natl Acad Sci U S A (1996) 93, 3428-33 ; Stroke, (1998) 29, 152-158; Stroke (1994) 25, 2265-2270).

La présente invention a donc pour objet des composés de formule générale (I) 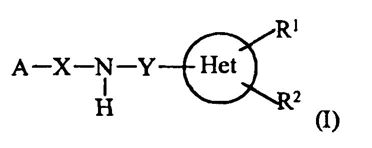
dans laquelle
R¹ représente un atome d'hydrogène, un radical -OR³, -SR³, oxo ou un acétal cyclique,
dans lequel R³ représente un atome d'hydrogène, un radical alkyle, arylalkyle, hétérocycloalkylcarbonyle, alkylcarbonyle, arylcarbonyle ou aralkylcarbonyle,
dans lesquels les radicaux alkyle, aryle ou hétérocycloalkyle sont éventuellement substitués par un ou plusieurs substituants identiques ou différents choisis parmi : alkyle, OH, alkoxy, nitro, cyano, halogène ou -NR⁴R⁵ ;
R⁴ et R⁵ représentent, indépendamment, un atome d'hydrogène ou un radical alkyle, ou bien R⁴ et R⁵ forment ensemble avec l'atome d'azote auquel ils sont rattachés un hétérocycle éventuellement susbtitué,
R² représente un atome d'hydrogène, un radical alkyle, aryle ou aralkyle, le groupement aryle étant éventuellement substitué par un ou plusieurs radicaux identiques ou différents choisis parmi : -OR⁶, -NR⁷R⁸, halogène, cyano, nitro ou alkyle,
dans lequel R⁶, R⁷ et R⁸ représentent, indépendamment, un atome d'hydrogène, un radical alkyle, aryle, aralkyle, alkylcarbonyle, arylcarbonyle ou aralkylcarbonyle;
A représente
un radical A1 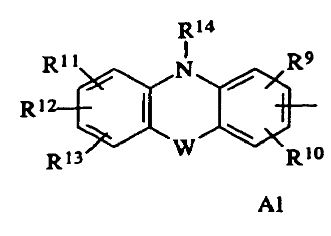
dans lequel R⁹, R¹⁰, R¹¹, R¹², R¹³ représentent, indépendamment, un atome d'hydrogène, un halogène, le groupe OH, un radical alkyle, alkoxy, cyano, nitro ou -NR¹⁵R¹⁶,
R¹⁵ et R¹⁶ représentent, indépendamment, un atome d'hydrogène, un radical alkyle ou un groupe -COR¹⁷, ou bien R¹⁵ et R¹⁶ forment ensemble avec l'atome d'azote auquel ils sont rattachés un hétérocycle éventuellement substitué,
R¹⁷ représente un atome d'hydrogène, un radical alkyle, alkoxy ou -NR¹⁸R¹⁹,
R¹⁸ et R¹⁹ représentent, indépendamment, un atome d'hydrogène ou un radical alkyle, ou bien R¹⁸ et R¹⁹ forment ensemble avec l'atome d'azote auquel ils sont rattachés un hétérocycle éventuellement substitué,
R¹⁴ représente un atome d'hydrogène, un radical alkyle ou un groupe -COR²⁰,
R²⁰ représente un atome d'hydrogène, un radical alkyle, alkoxy, aryle, aralkyle, hétérocycloalkyle ou -NR²¹R²²,
dans lesquels les radicaux alkyle, aryle ou hétérocycloalkyle sont éventuellement substitués par un ou plusieurs substituants identiques ou différents choisis parmi : alkyle, OH, alkoxy, nitro, cyano, halogène ou -NR⁴R⁵;
R²¹ et R²² représentent, indépendamment, un atome d'hydrogène ou un radical alkyle, ou bien R²¹ et R²² forment ensemble avec l'atome d'azote auquel ils sont rattachés un hétérocycle éventuellement substitué,
W représente une liaison, O ou S ou encore un radical -NR²³, dans lequel R²³ représente un atome d'hydrogène ou un radical alkyle ;
X représente -(CH₂)ₙ-, -(CH₂)ₙ-CO-, -N(R⁴⁵)-CO-(CH₂)ₙ-CO-, -N(R⁴⁵)-CO-D-CO-, -CO-N(R⁴⁵)-D-CO-, -CO-D-CO-, -CH=CH-(CH₂)ₙ-CO-, -N(R⁴⁵)-(CH₂)ₙ-CO-, -N(R⁴⁵)-CO-C(R⁴⁶R⁴⁷)-CO-, -O-(CH₂)ₙ-CO-, -N(R⁴⁵)-CO-NH-C(R⁴⁶R⁴⁷)-CO-, -CO-N(R⁴⁵)-C(R⁴⁶R⁴⁷)-CO-, -S-(CH2)ₙ-CO- ou -Z-CO- ;
D représente un radical phénylène éventuellement substitué par un ou plusieurs radicaux identiques ou différents choisis parmi alkyle, alkoxy, OH, nitro, halogène, cyano, ou carboxyl éventuellement estérifié par un radical alkyle ;
Z représente un hétérocycle,
R⁴⁵ représente un atome d'hydrogène ou un radical alkyle,
R⁴⁶ et R⁴⁷ représentent, indépendamment, un atome d'hydrogène, un radical alkyle, aryle ou aralkyle dont les groupements alkyle et aryle sont éventuellement substitués par un ou plusieurs substituants identiques ou différents choisis parmi : le groupe OH, -SH, halogène, nitro, alkyle, alkoxy, alkylthio, aralkoxy, aryl-alkylthio, -NR⁴⁸R⁴⁹ et carboxyl éventuellement estérifié par un radical alkyle ;
R⁴⁸ et R⁴⁹ représentent, indépendamment, un atome d'hydrogène, un radical alkyle ou un groupe -COR⁵⁰, ou bien R⁴⁸ et R⁴⁹ forment ensemble avec l'atome d'azote auquel ils sont rattachés un hétérocycle éventuellement substitué,
R⁵⁰ représente un atome d'hydrogène, un radical alkyle, alkoxy ou -NR⁵¹R⁵²,
R⁵¹ et R⁵² représentent, indépendamment, un atome d'hydrogène ou un radical alkyle, ou bien R⁵¹ et R⁵² forment ensemble avec l'atome d'azote auquel ils sont rattachés, un hétérocycle éventuellement substitué ;
n étant un entier compris entre 0 et 6 ;
Y représente -(CH₂)ₚ- , -C(R⁵³R⁵⁴)-(CH₂)ₚ- , -C(R⁵³R⁵⁴)-CO- ;
R⁵³ et R⁵⁴ représentent, indépendamment, un atome d'hydrogène, un radical alkyle, un radical aralkyle dont le groupement aryle est éventuellement substitué par un ou plusieurs substituants identiques ou différents choisis parmi : le groupe OH, halogène, nitro, alkyle, alkoxy, -NR⁵⁵R⁵⁶,
R⁵⁵ et R⁵⁶ représentent, indépendamment, un atome d'hydrogène, un radical alkyle ou un groupe -COR⁵⁷, ou bien R⁵⁵ et R⁵⁶ forment ensemble avec l'atome d'azote auquel ils sont rattachés, un hétérocycle éventuellement substitué,
R⁵⁷ représente un atome d'hydrogène, un radical alkyle, alkoxy ou -NR⁵⁸R⁵⁹,
R⁵⁸ et R⁵⁹ représentent, indépendamment, un atome d'hydrogène ou un radical alkyle, ou bien R⁵⁸ et R⁵⁹ forment ensemble avec l'atome d'azote auquel ils sont rattachés un hétérocycle éventuellement substitué ;
p étant un entier compris entre 0 et 6 ;
Het représente le radical tétrahydrofuran-3-yl,
ainsi que les sels d'addition avec les acides minéraux et organiques ou avec les bases minérales et organiques desdits composés de formule générale (I),
à l'exclusion des composés de formule (I) dans laquelle lorsque Het représente tétrahydrofuranne ou tétrahydropyranne, R¹ le radical OR³ avec R³ représentant un atome d'hydrogène, un radical alkyle, arylalkyle, hétérocycloalkylcarbonyle dont le radical hétérocycloalkyl est branché par un atome de carbone, alkylcarbonyle, arylcarbonyle ou aralkylcarbonyle, R² un hydrogène et Y le radical -(CH₂)ₚ- avec p = 0, alors X représente -CO-N(R⁴⁵)-C(R⁴⁶R⁴⁷)-CO- avec R⁴⁵ = R⁴⁶ = H.

Dans certains cas, les composés selon la présente invention peuvent comporter des atomes de carbone asymétriques (de configuration "R" ou "S"). Par conséquent, la présente invention inclut les formes énantiomères, diastéréoisomères et toutes combinaisons de ces formes, y compris les mélanges racémiques "RS". Dans un souci de simplicité, lorsqu'aucune configuration spécifique n'est indiquée dans les formules de structure, il faut comprendre que les deux formes énantiomères (ou diastéréoisomères) et leurs mélanges sont représentés.

Par alkyle, lorsqu'il n'est pas donné plus de précision, on entend un radical alkyle linéaire ou ramifié comptant de 1 à 6 atomes de carbone comme, par exemple, les radicaux méthyle, éthyle, propyle, isopropyle, butyle, isobutyle, sec-butyle et tert-butyle, pentyle, néopentyle, isopentyle, hexyle, isohexyle. Les radicaux alkoxy peuvent correspondre aux radicaux alkyle indiqués ci-dessus comme par exemple les radicaux méthoxy, éthoxy, propyloxy ou isopropyloxy mais également butoxy linéaire, secondaire ou tertiaire. De même les radicaux alkylthio peuvent correspondre aux radicaux alkyle indiqués ci-dessus comme par exemple méthylthio ou éthylthio.

Par alkényle, lorsqu'il n'est pas donné plus de précision, on entend un radical alkyle linéaire ou ramifié comptant de 1 à 6 atomes de carbone et présentant au moins une insaturation (double liaison). Par halogène, on entend les atomes de fluor, de chlore, de brome ou d'iode.

Par aryle, on entend un système carbocyclique ou hétérocyclique comprenant au moins un cycle aromatique, un système étant dit hétérocyclique lorsque l'un au moins des cycles qui le composent comporte un hétéroatome (O, N ou S). Comme exemple de radical aryle carbocyclique, on peut citer phényle ou naphtyle. Comme exemple de radical aryle hétérocyclique (ou hétéroaryle), on peut citer thiényle, furyle, pyrrolyle, imidazolyle, pyrazolyle, isothiazolyle, thiazolyle, isoxazolyle, oxazolyle, pyridyle, pyrazinyle, pyrimidyle, benzothiényle, benzofuryle et indolyle.

Le terme hétérocycle (ou hétérocycloalkyle), représenté par exemple par les radicaux Het ou Z, représente de préférence un hétérocycle mono ou bicyclique, saturé ou insaturé, comportant de 1 à 5 hétéroatomes choisis parmi O, S, N. L'atome d'azote peut éventuellement être substitué par un radical choisi parmi : alkyle, aryle, aralkyle et alkylcarbonyle. Comme exemple d'hétérocycle saturé, on peut citer : tétrahydrofuranne, tétrahydropyranne, oxétane, oxépane, tétrahydrothiophène, tétrahydrothiopyranne, thiétane, pyrrolidine, pipéridine, azétidine, 1,3-dioxanne, 1,3-dioxolanne, 1,3-dithiolanne, 1,3-dithianne, 1,3-oxathiolanne, 1,3-oxazolidine, 1,3-imidazolidine ou 1,3-thiazolidine. Comme exemple d'hétérocycle insaturé, on peut citer : thiophène, furane, pyrrole, imidazole, pyrazole, isothiazole, thiazole, isoxazole, oxazole, pyridine, pyrazine, pyrimidine, benzimidazole, benzofuranne, benzopyranne, 1,3-benzothiazole, benzoxazole, quinoléine.

Les radicaux arylalkyles (ou aralkyles) désignent les radicaux dans lesquels respectivement les radicaux aryle et alkyle sont tels que définis ci-dessus comme par exemple benzyle, phenéthyle ou naphtylméthyle. Les radicaux aralkoxy (aryl-alkoxy) désignent les radicaux dans lesquels respectivement les radicaux aryle et alkoxy sont tels que définis ci-dessus comme par exemple benzyloxy ou phényléthoxy. Les radicaux arylalkylthio désignent les radicaux dans lesquels respectivement les radicaux aryle et alkylthio sont tels que définis ci-dessus comme par exemple benzylthio.

Les radicaux alkylcarbonyle, hétérocycloalkylcarbonyle, arylcarbonyle ou aralkylcarbonyle désignent les radicaux dans lesquels respectivement les radicaux alkyle, hétérocycloalkyle, aryle et aralkyle ont la signification indiquée précédemment.

Dans le cas de radicaux de formule -NRⁱR^{j} où Rⁱ et R^{j} forment ensemble avec l'atome d'azote auquel ils sont rattachés un hétérocycle éventuellement substitué, l'hétérocycle est de préférence saturé et comprend de 4 à 7 chaînons et de 1 à 3 hétéroatomes incluant l'atome d'azote déjà présent, les hétéroatomes supplémentaires étant choisis indépendamment dans le groupe constitué des atomes O, N et S. Ledit hétérocycle peut être, par exemple, le cycle azétidine, pyrrolidine, pipéridine, pipérazine, morpholine ou thiomorpholine. Ledit hétérocycle peut être substitué par un ou plusieurs substituants identiques ou différents choisis parmi le groupe hydroxy, un radical alkyle, aryle, aralkyle ou alkoxy ou un atome d'halogène.

L'invention a plus particulièrement pour objet des composés de formule (I) telle que définie ci-dessus, dans laquelle R¹ représente l'atome d'hydrogène, le radical -OR³ ou oxo.

L'invention a plus particulièrement pour objet également des composés de formule (I) telle que définie ci-dessus, dans laquelle X représente -(CH2)ₙ-, -(CH₂)ₙ-CO-, -O-(CH₂)ₙ-CO-, -CO-N(R⁴⁵)-D-CO-, -N(R⁴⁵)-CO-(CH₂)ₙ-CO-, -N(R⁴⁵)-CO-C(R⁴⁶R⁴⁷)-CO-, -N(R⁴⁵)-CO-NH-C (R⁴⁶R⁴⁷)-CO-, -N(R⁴⁵)-(CH₂)ₙ -CO-, -CO-N(R⁴⁵)-C(R⁴⁶R⁴⁷)-CO- ou -Z-CO-,
et préférentiellement lorsque R⁴⁵ et R⁴⁷ représentent l'atome d'hydrogène, R⁴⁶ l'atome d'hydrogène, un radical alkyle ou phényle, D le radical phénylène et Z le radical thiazole.

L'invention a plus particulièrement pour objet également des composés de formule (I) telle que définie ci-dessus, dans laquelle R² représente un atome d'hydrogène ou un radical aralkyle, et de préférence le radical benzyle.

L'invention a plus particulièrement pour objet également des composés de formule (I) telle que définie ci-dessus, dans laquelle A représente
A1 avec W représentant l'atome de soufre,
et de manière préférentielle le radical de formule

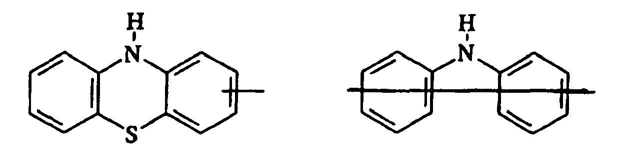

Plus particulièrement également, l'invention a pour objet des composés décrits ci-après dans les exemples et préférentiellement les produits répondant aux formules suivantes :
N-[(1S)-1-([(3S)-2-hydroxytétrahydro-3-furanyl]amino}carbonyl)-3-méthylbutyl]-10*H-*phénothiazine-2-carboxamide ;
Acétate de (3*S*)-3-({(2*S*)-4-méthyl-2-[(10*H*-phénothiazin-2-ylcarbonyl)-amino]pentanoyl}amino)tétrahydro-2-furanyl ;
N-[(3S)-2-hydroxytétrahydro-3-furanyl)-2-(10*H*-phénothiazin-2-yl)-1,3-thiazole-4-carboxamide ;
N-[4-({[(3S)-2-hydroxytétrahydro-3-furanyl]amino}carbonyl)phenyl]-10*H-*phénothiazine-2-carboxamide ;
N-[(1S)-1-({[(3S)-2-hydroxytétrahydro-3-furanyl]amino}carbonyl)-3-méthylbutyl]-10*H*-phénothiazine-1-carboxantide ;
Pivalate de (3S)-3-({(2S)-4-méthyl-2-[(10*H*-phénothiazin-2-ylcarbonyl)amino] pentanoyl}amino)tétrahydro-2-furanyl ;
3,3-diméthylbutanoate de (3S)-3-({(2S)-4-méthyl-2-[(10*H*-phénothiazin-2-ylcarbonyl) amino]pentanoyl}amino)tétrahydro-2-furanyl ;
(3S)-3-({2S)-4-méthyl-2-[(10*H*-phénothiazin-2-ylcarbonyl)amino]pentanoyl}amino) tétrahydro-2-furanyl benzoate ;
(3S)-3-({(2S)-4-méthyl-2-[(10*H*-phénothiazin-2-ylcarbonyl)amino]pentanoyl}amino) tétrahydro-2-furanyl phénylacetate ;
(3*S*)-3-({(2*S*)-4-méthyl-2-[(10*H*-phénothiazin-2-ylcarbonyl)amino]pentanoyl}amino) tétrahydro-2-furanyl (2*S*)-2-(diméthylamino)-3-phénylpropanoate ;
4-morpholinecarboxylate de (3*S*)-3-({(2*S*)-4-méthyl-2-[(10*H*-phénothiazin-2-ylcarbonyl) amino]pentanoyl}amino)tétrahydro-2-furanyl ;

L'invention a plus particulièrement pour objet également les composés répondant à l'une des formules suivantes :
N-[(1S)-1-({[(3S)-2-hydroxytétrahydro-3-furanyl]amino}carbonyl)-3-méthylbutyl]-10H-phénothiazine-2-carboxamide ;
Acétate de (3*S*)-3-({(2*S*)-4-méthyl-2-[(10*H*-phénothiazin-2-ylcarbonyl)-amino] pentanoyl}amino)tétrahydro-2-furanyl ;
Acétate de (2R,3S)-3-({(2S)-4-méthyl-2-[(10H-phénothiazin-2-ylcarbonyl)amino]-pentanoyl}amino)tétrahydro-2-furanyl ;
Acétate de (2S,3S)-3-({(2S)-4-méthyl-2-[(10H-phénothiazin-2-ylcarbonyl) amino]-pentanoyl}amino)tétrahydro-2-furanyl ; ainsi que les sels d'addition avec les acides minéraux et organiques ou avec les bases minérales et organiques desdits composés.

Les composés de formule I selon l'invention peuvent être préparés suivant plusieurs voies de synthèse selon la définition des groupes variables.

Les composés de formule générale (I) dans laquelle Het représente le cycle tétrahydrofuranne et Y le radical -(CH₂)ₚ, peuvent être préparés selon le schéma suivant : 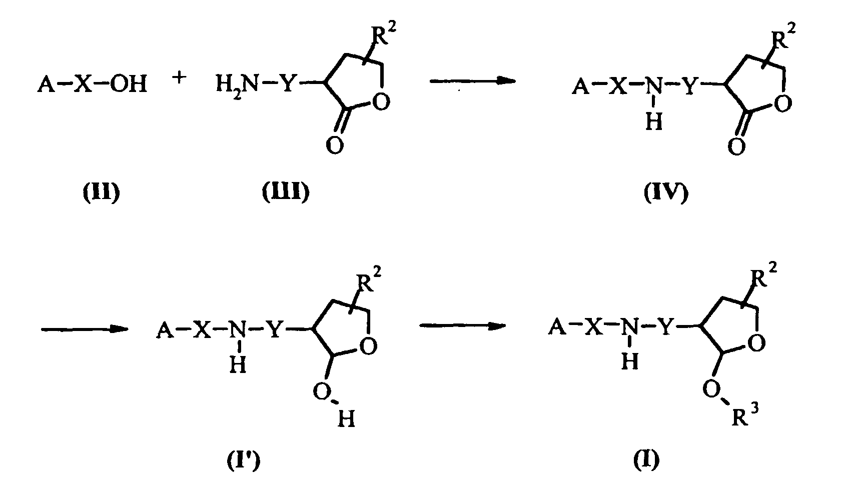
dans lesquels A, X, R² et R³ sont tels que décrits ci-dessus,
par condensation des acides de formule générale (II) sur les amines de formule générale (III), dans les conditions classiques de la synthèse peptidique (M. Bodanszky et A. Bodanszky, The Practice of Peptide Synthesis, 145 (Springer-Verlag, 1984)) dans le THF, le dichlorométhane ou le DMF en présence d'un réactif de couplage tels que le dicyclohexylcarbodiimide (DCC), le 1,1'-carbonyldiimidazole (CDI) (J. Med. Chem. (1992), 35 (23), 4464-4472) ou le chlorhydrate de 1-(3-diméthylaminopropyl)-3-éthylcarbodiimide (EDC ou WSCI) (John Jones, The chemical synthesis of peptides, 54 (Clarendon Press, Oxford, 1991)) pour conduire aux carboxamides intermédiaires de formule générale (IV). Le cycle lactonique des intermédiaires de formule générale (IV) est ensuite réduit à l'aide d'un agent réducteur tel que, par exemple, l'hydrure de Diisobutylaluminium (DIBAL), dans un solvant inerte tel que, par exemple, THF ou CH₂Cl₂, à une température variant de 0 à -78° C. Le dérivé lactol de formule générale (I') ainsi obtenu peut être acylé à l'aide, par exemple, d'un chlorure d'acide (R³-Cl) ou d'un anhydride d'acide (anhydride acétique, chlorure de benzoyle, ...) en présence d'une base telle que, par exemple, la triéthylamine, dans un solvant inerte comme par exemple CH₂Cl₂ pour conduire au composé de formule générale (I).

Les composés de formule générale (I) dans laquelle Het représente le cycle tétrahydrofuranne, X le radical -(CH₂)ₙ- (n = 0) et Y le radical -(CH₂)ₚ- (p = 0), peuvent également être préparés selon le schéma suivant : 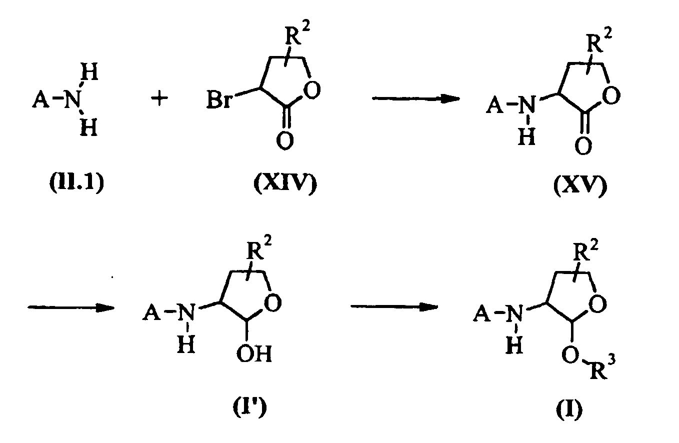
dans lesquels A, R² et R³ sont tels que décrits ci-dessus,
par substitution nucléophile de l'halogène des lactones de formule générale (XV) à l'aide des amines de formule générale (II.1), en chauffant le mélange réactionnel à une température variant de 50 à 110° C dans un solvant inerte tel que par exemple, l'acétonitrile ou le DMF, pendant une durée variant de 30 minutes à 5 heures, pour conduire aux intermédiaires de formule générale (XV). La réduction de la fonction lactone suivie de l'acylation du lactol de formule générale (I') sont effectuées dans les conditions précédemment décrites.

Les composés de formule générale (I) dans laquelle Het représente le cycle tétrahydrofuranne, X le radical -N(R⁴⁵)-(CH₂)ₙ-CO- (n = 0) et Y le radical -(CH₂)ₚ- (p = 0) sont des urées qui peuvent être préparées selon le schéma synthétique suivant : 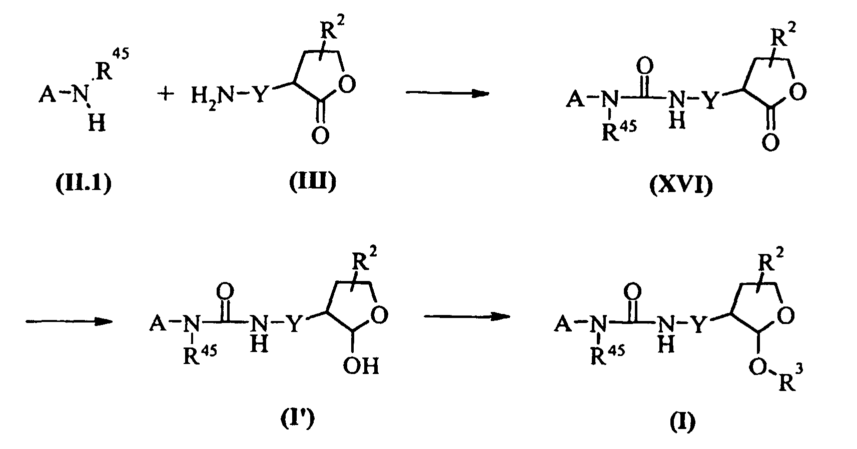
dans lesquels A, R² et R³ sont tels que décrits ci-dessus,
par condensation des amines de formule générale (II.1) avec les amines de formule générale (III) en présence de triphosgène et d'une base telle que, par exemple, la diisopropyléthylamine dans un solvant inerte tel que le dichlorométhane selon un protocole expérimental décrit dans J. Org. Chem. (1994) 59 (7), 1937-1938. Le cycle lactonique des urées de formule générale (XVI) est ensuite réduit et modifié dans les conditions expérimentales précédemment décrites pour conduire aux composés de formule générale (I).

Les composés de la présente invention possèdent d'intéressantes propriétés pharmacologiques : ils présentent une activité inhibitrice des calpaïnes et / ou une activité piégeuse des formes réactives de l'oxygène.

Les composés de la présente invention peuvent ainsi être utilisés dans différentes applications thérapeutiques. Ils peuvent produire des effets bénéfiques ou favorables dans le traitement de pathologies où ces enzymes et/ou ces espèces radicalaires sont impliquées.

Ces propriétés rendent les produits de formule I aptes à une utilisation pharmaceutique. La présente demande a également pour objet, à titre de médicaments, les produits de formule I telle que définie ci-dessus, ainsi que les sels d'addition avec les acides minéraux ou organiques ou les bases minérales et organiques pharmaceutiquement acceptables desdits produits de formule I, ainsi que les compositions pharmaceutiques contenant, à titre de principe actif, l'un au moins des médicaments tels que définis ci-dessus.

L'invention concerne ainsi des compositions pharmaceutiques contenant un composé de l'invention ou un sel additif d'acide pharmaceutiquement acceptable de celui-ci, en association avec un support pharmaceutiquement acceptable. La composition pharmaceutique peut être sous forme d'un solide, par exemple, des poudres, des granules, des comprimés, des gélules ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau, additionnés à des huiles ou des graisses pharmaceutiquement acceptables. Les compositions liquides stériles peuvent être utilisées pour les injections intramusculaires, intrapéritonéales ou sous-cutanées et les compositions stériles peuvent également être administrées par intraveineuse.

Certains composés de la formule générale I précédemment décrite, sont couverts par la demande EP 641800. Les composés de cette demande présentent une activité inhibitrice de cathepsine L qui est différente de l'activité inhibitrice des calpaïnes et / ou de l'activité piégeuse des formes réactives de l'oxygène.

L'invention a donc également pour objet l'utilisation de composés de formule (Iₐ) telle que définie ci-dessus, 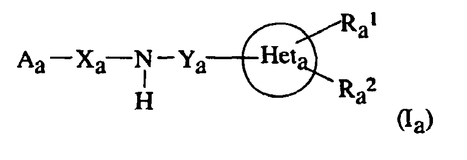
sous forme racémique, d'énantiomères, de diastéréoisomères ou toutes combinaisons de ces formes, dans laquelle
Rₐ¹ représente un atome d'hydrogène, un radical -OR³, -SR³, oxo ou un acétal cyclique,
dans lequel R³ représente un atome d'hydrogène, un radical alkyle, arylalkyle, hétérocycloalkylcarbonyle, alkylcarbonyle, arylcarbonyle ou aralkylcarbonyle,
dans lesquels les radicaux alkyle, aryle ou hétérocycloalkyle sont éventuellement substitués par un ou plusieurs substituants identiques ou différents choisis parmi : alkyle, OH, alkoxy, nitro, cyano, halogène ou -NR⁴R⁵;
R⁴ et R⁵ représentent, indépendamment, un atome d'hydrogène ou un radical alkyle, ou bien R⁴ et R⁵ forment ensemble avec l'atome d'azote auquel ils sont rattachés un hétérocycle éventuellement susbtitué,
Rₐ² représente un atome d'hydrogène, un radical alkyle, aryle ou aralkyle, le groupement aryle étant éventuellement substitué par un ou plusieurs radicaux identiques ou différents choisis parmi : -OR⁶, -NR⁷R⁸, halogène, cyano, nitro ou alkyle,
dans lequel R⁶, R⁷ et R⁸ représentent, indépendamment, un atome d'hydrogène, un radical alkyle, aryle, aralkyle, alkylcarbonyle, arylcarbonyle ou aralkylcarbonyle ;
Aₐ représente
un radical A1 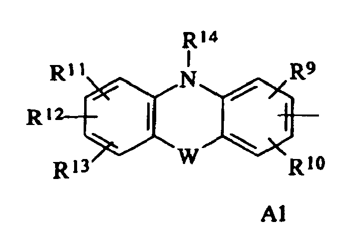
dans lequel R⁹, R¹⁰, R¹¹, R¹², R¹³ représentent, indépendamment, un atome d'hydrogène, un halogène, le groupe OH, un radical alkyle, alkoxy, cyano, nitro ou -NR¹⁵R¹⁶,
R¹⁵ et R¹⁶ représentent, indépendamment, un atome d'hydrogène, un radical alkyle ou un groupe -COR¹⁷, ou bien R¹⁵ et R¹⁶ forment ensemble avec l'atome d'azote auquel ils sont rattachés un hétérocycle éventuellement substitué,
R¹⁷ représente un atome d'hydrogène, un radical alkyle, alkoxy ou -NR¹⁸R¹⁹,
R¹⁸ et R¹⁹ représentent, indépendamment, un atome d'hydrogène ou un radical alkyle, ou bien R¹⁸ et R¹⁹ forment ensemble avec l'atome d'azote auquel ils sont rattachés un hétérocycle éventuellement substitué,
R¹⁴ représente un atome d'hydrogène, un radical alkyle ou un groupe -COR²⁰,
R²⁰ représente un atome d'hydrogène, un radical alkyle, alkoxy, aryle, aralkyle, hetérocycloalkyle ou -NR²¹R²²,
dans lesquels les radicaux alkyle, aryle ou hétérocycloalkyle sont éventuellement substitués par un ou plusieurs substituants identiques ou différents choisis parmi : alkyle, OH, alkoxy, nitro, cyano, halogène ou -NR⁴R⁵;
R²¹ et R²² représentent, indépendamment, un atome d'hydrogène ou un radical alkyle, ou bien R²¹ et R²² forment ensemble avec l'atome d'azote auquel ils sont rattachés un hétérocycle éventuellement substitué,
W représente une liaison, O ou S ou encore un radical -NR²³, dans lequel R²³ représente un atome d'hydrogène ou un radical alkyle ;
Xₐ représente -(CH₂)ₙ-, -(CH₂)ₙ-CO-, -N(R⁴⁵)-CO-(CH₂)ₙ-CO-, -N(R⁴⁵)-CO-D-CO-, -CO-N(R⁴⁵)-D-CO-, -CO-D-CO-, -CH=CH-(CH₂)ₙ-CO-, -N(R⁴⁵)-(CH₂)ₙ-CO-, -N(R⁴⁵)-CO-C(R⁴⁶R⁴⁷)-CO-, -O-(CH₂)ₙ-CO-, -N(R⁴⁵)-CO-NH-C(R⁴⁶R⁴⁷)-CO-, -CO-N(R⁴⁵)-C(R⁴⁶R⁴⁷)-CO-, -S-(CH₂)ₙ-CO- ou -Z-CO- ;
D représente un radical phénylène éventuellement substitué par un ou plusieurs radicaux identiques ou différents choisis parmi alkyle, alkoxy, OH, nitro, halogène, cyano, ou carboxyl éventuellement estérifié par un radical alkyle ;
Z représente un hétérocycle,
R⁴⁵ représente un atome d'hydrogène ou un radical alkyle,
R⁴⁶ et R⁴⁷ représentent, indépendamment, un atome d'hydrogène, un radical alkyle, aryle ou aralkyle dont les groupements alkyle et aryle sont éventuellement substitués par un ou plusieurs substituants identiques ou différents choisis parmi : le groupe OH, -SH, halogène, nitro, alkyle, alkoxy, alkylthio, aralkoxy, aryl-alkylthio, -NR⁴⁸R⁴⁹ et carboxyl éventuellement estérifié par un radical alkyle ;
R⁴⁸ et R⁴⁹ représentent, indépendamment, un atome d'hydrogène, un radical alkyle ou un groupe -COR⁵⁰, ou bien R⁴⁸ et R⁴⁹ forment ensemble avec l'atome d'azote auquel ils sont rattachés un hétérocycle éventuellement substitué,
R⁵⁰ représente un atome d'hydrogène, un radical alkyle, alkoxy ou -NR⁵¹R⁵²,
R⁵¹ et R⁵² représentent, indépendamment, un atome d'hydrogène ou un radical alkyle, ou bien R⁵¹ et R⁵² forment ensemble avec l'atome d'azote auquel ils sont rattachés, un hétérocycle éventuellement substitué ;
n étant un entier compris entre 0 et 6 ;
Yₐ représente -(CH₂)ₚ- , -C(R⁵³R⁵⁴)-(CH₂)ₚ-, -C(R⁵³R⁵⁴)-CO- ;
R⁵³ et R⁵⁴ représentent, indépendamment, un atome d'hydrogène, un radical alkyle, un radical aralkyle dont le groupement aryle est éventuellement substitué par un ou plusieurs substituants identiques ou différents choisis parmi : le groupe OH, halogène, nitro, alkyle, alkoxy, -NR⁵⁵R⁵⁶,
R⁵⁵ et R⁵⁶ représentent, indépendamment, un atome d'hydrogène, un radical alkyle ou un groupe -COR⁵⁷, ou bien R⁵⁵ et R⁵⁶ forment ensemble avec l'atome d'azote auquel ils sont rattachés, un hétérocycle éventuellement substitué,
R⁵⁷ représente un atome d'hydrogène, un radical alkyle, alkoxy ou -NR⁵⁸R⁵⁹,
R⁵⁸ et R⁵⁹ représentent, indépendamment, un atome d'hydrogène ou un radical alkyle, ou bien R⁵⁸ et R⁵⁹ forment ensemble avec l'atome d'azote auquel ils sont rattachés un hétérocycle éventuellement substitué ;
p étant un entier compris entre 0 et 6 ;
Hetₐ représente le radical tétrahydrofuran-3-yl,
ainsi que des sels d'addition avec les acides minéraux et organiques ou avec les bases minérales et organiques desdits composés de formule générale (I),
pour la préparation de médicaments pour le traitement de pathologies où les calpaïnes et / ou les formes réactives de l'oxygène sont impliquées.

L'invention a plus particulièrement pour objet l'utilisation de composés de formule (Iₐ) telle que définie ci-dessus, pour la préparation de médicaments pour le traitement de pathologies où les formes réactives de l'oxygène sont impliquées. L'invention a plus particulièrement pour objet également l'utilisation de composés de formule (Iₐ) telle que définie ci-dessus, pour la préparation de médicaments pour le traitement de pathologies où les formes réactives de l'oxygène et les calpaïnes sont impliquées. L'invention concerne donc l'utilisation de composés de formule (Iₐ) telle que définie ci-dessus, pour la préparation de médicaments pour le traitement de pathologies comme les maladies inflammatoires et immunologiques, les maladies cardio-vasculaires et cérébro-vasculaires, les troubles du système nerveux central ou phériphérique, l'ostéoporose, les dystrophies musculaires, les maladies prolifératives, la cataracte, les transplantations d'organes, les maladies auto-immunes et virales, le cancer, et toutes les pathologies caractérisées par une production excessive des ROS et / ou une activation des calpaïnes.

L'invention a plus particulièrement pour objet l'utilisation de composés de formule (Iₐ) telle que définie ci-dessus, caractérisée en ce que R¹ représente l'atome d'hydrogène, le radical -OR³ ou oxo.

L'invention a plus particulièrement pour objet l'utilisation de composés de formule (Iₐ) telle que définie ci-dessus, caractérisée en ce que X représente -(CH₂)ₙ-, -(CH₂)ₙ-CO-, -O-(CH₂)ₙ-CO-, -CO-N(R⁴⁵)-D-CO-, -Z-CO-, -N(R⁴⁵)-CO-(CH₂)ₙ-CO-, -N(R⁴⁵)-CO-C(R⁴⁶R⁴⁷)-CO, -N(R⁴⁵)-(CH₂)ₙ-CO-, -N(R⁴⁵)-CO-NH-C(R⁴⁶R⁴⁷)-CO- ou -CO-N(R⁴⁵)-C(R⁴⁶R⁴⁷)-CO et préférentiellement lorsque R⁴⁵ et R⁴⁷ représentent l'atome d'hydrogène, R⁴⁶ l'atome d'hydrogène, un radical alkyle ou phényle, D le radical phénylène et Z le radical thiazole.

L'invention a plus particulièrement pour objet l'utilisation de composés de formule (Iₐ) telle que définie ci-dessus caractérisée en ce que R² représente un atome d'hydrogène ou un radical aralkyle, et de préférence le radical benzyle.

L'invention a plus particulièrement pour objet l'utilisation de composés de formule (Iₐ) telle que définie ci-dessus caractérisée en ce que A représente A1 avec W représentant l'atome de soufre. De manière préférentielle A représente le radical

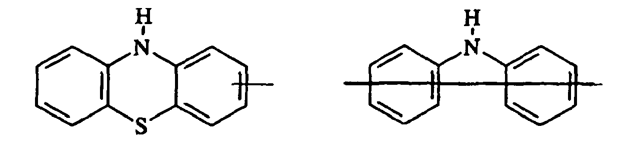

Plus particulièrement également, l'invention a pour objet l'utilisation telle que définie ci-dessus, de composés de formule (Iₐ) tels que décrits dans les exemples et préférentiellement les composés qui répondent à l'une des formules suivantes :
N-[(1S)-1-({[(3S)-2-hydroxytétrahydro-3-furanyl]amino}carbonyl)-3-méthylbutyl)-10H-phénothiazine-2-carboxamide ;
Acétate de (3*S*)-3-({(2*S*)-4-méthyl-2-[(10*H*-phénothiazin-2-ylcarbonyl)-amino]pentanoyl}amino)tétrahydro-2-furanyl ;
N-[(3S)-2-hydroxytétrahydro-3-furanyl]-2-(10*H*-phénothiazin-2-yl)-1,3-thiazole-4-carboxamide ;
N-[4-({[(3S)-2-hydroxytétrahydro-3-furanyl]amino}carbonyl)phényl]-10*H-*phénothiazine-2-carboxamide ;
N-[(1S)-1-({[(3S)-2-hydroxytétrahydro-3-furanyl]amino}carbonyl)-3-méthylbutyl]-10*H*-phénothiazine-1-carboxamide ;
Pivalate de (3S)-3-({(2S)-4-méthyl-2-[(10*H*-phénothiazin-2-ylcarbonyl)amino] pentanoyl}amino)tétrahydro-2-furanyl ;
3,3-diméthylbutanoate de (3S)-3-({(2S)-4-méthyl-2-[(10*H*-phénothiazin-2-ylcarbonyl) amino]pentanoyl}amino)tétrahydro-2-furanyl ;
(3S)-3-({(2S)-4-méthyl-2-[(10*H*-phénothiazin-2-ylcarbonyl)amino]pentanoyl}amino) tétrahydro-2-furanyl benzoate ;
(3S)-3-({(2S)-4-méthyl-2-[(10*H*-phénothiazin-2-ylcarbonyl)amino]pentanoyl} amino) tétrahydro-2-furanyl phénylacetate ;
(3*S*)-3-({(2*S*)-4-méthyl-2-[(10*H*-phénothiazin-2-ylcarlxmyl)amino]pentanoyl} amino) tétrahydro-2-furanyl (2*S*)-2-(diméthylamino)-3-phénylpropanoate ;
4-morpholinecarboxylate de (3S)-3-({(2S)-4-méthyl-2-((10*H*-phénothiazin-2-ylcarbonyl) amino]pentanoyl}amino)tétrahydro-2-furanyl ;

L'invention a plus particulièrement pour objet également l'utilisation de composés de formule (Iₐ) telles que définies ci-dessus et caractérisés en ce qu'ils répondent à l'une des formules suivantes :
N-[(1S)-1-({[(3S)-2-hydroxytétrahydro-3-furanyl]amino}carbonyl)-3-méthylbutyl]-10H-phénothiazine-2-carboxamide ;
Acétate de (3*S*)-3-({(2*S*)-4-méthyl-2-[(10*H*-phénothiazin-2-ylcarbonyl)-amino] pentanoyl}amino)tétrahydro-2-furanyl ;
Acétate de (2R,3S)-3-({(2S)-4-méthyl-2-[(10H-phénothiazin-2-ylcarbonyl)amino]-pentanoyl}amino)tétrahydro-2-furanyl ;
Acétate de (2S,3S)-3-({(2S)-4-méthyl-2-[(10H-phénothiazin-2-ylcarbonyl)amino]-pentanoyl}amino)tétrahydro-2-furanyl.

Les intermédiaires de synthèse non commerciaux de formule (II), (III) et (V) peuvent être préparés selon les différentes voies de synthèse ci-dessous :

### 1) Synthèse des intermédiaires (II) :

Les acides carboxyliques de formule générale (II), dans lesquels A, X, D, n, R⁴⁵, R⁴⁶ et R⁴⁷ sont tels que décrits ci-dessus, sont accessibles à partir des schémas synthétiques suivants :

### 1.1) A partir de A-NH(R⁴⁵) :

La préparation des acides carboxyliques de formule générale (II) peut être effectuée, dans ce cas, à partir de 3 dérivés acides-esters différents (II.2), (II.A) et (II.6) :

La condensation des anilines de formule générale (II.1) avec les acide-esters (Alk = Alkyle) commerciaux de formule générale (II.2), schéma 1.1, est effectuée par condensation peptidique classique. Le carboxamide intermédiairement obtenu (II.3) est ensuite saponifié pour conduire aux acides carboxyliques de formule générale (II). La synthèse des intermédiaires de formule générale (II.1) est décrite plus loin.

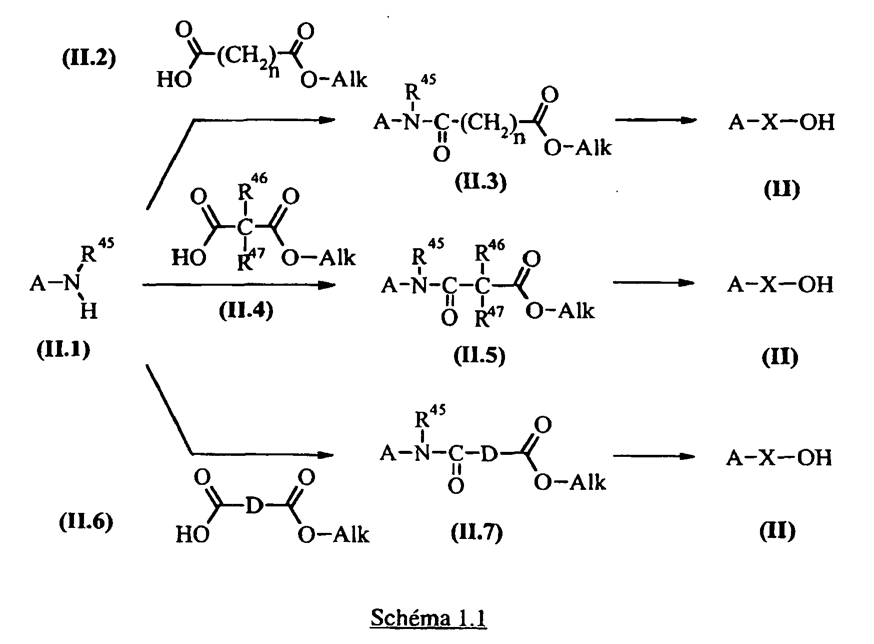

La synthèse des acides carboxyliques de formule générale (II) peut également être effectuée par condensation des anilines de formule générale (II.1) avec les dérivés d'acide-esters de formule générale (II.4) dans les conditions précédemment décrites. Cette condensation est suivie d'une saponification classique pour conduire aux acides de formule générale (II). La synthèse des intermédiaires de formule générale (II.4) est décrite plus loin.

La condensation des amines de formule générale (II.1) avec les acides aromatiques commerciaux de formule générale (II.6), dans les conditions de synthèse peptidique déjà décrites, permet après saponification des intermédiaires de formule générale (II.7) d'obtenir également les acides carboxyliques de formule générale (II).

Alternativement les acides carboxyliques de formule générale (II), sont également accessibles par l'ouverture d'anhydrides cycliques tels que par exemple, l'anhydride succinique, à l'aide des amines de formule générale (II.1) selon un protocole expérimental décrit dans la littérature (J. Amer. Chem. Soc. (1951) 73, 4007).

### 1.1.1) Préparation des intermédiaires (II.1) :

Les anilines de formule générale (II.1), non commerciales, dérivés d'indoline ou de 1,2,3,4-tétrahydroquinoline, schéma 1.1.1, dans lesquelles T et R³⁸ sont tels que définis ci-dessus, peuvent être préparées à partir des dérivés nitro correspondants de formule générale (II.1.1). La 6-nitro-1,2,3,4-tétrahydroquinoline est décrite dans Can. J. Chem. (1952), 30, 720-722. L'alkylation de l'amine est classiquement effectuée par une base forte telle que, par exemple, NaH, dans un solvant aprotique polaire tel que, par exemple, le DMF en présence d'un dérivé halogéné R³⁸-Hal, tels que par exemple, le chlorure de 3-diméthylaminopropane ou le bromure de benzyle. Le dérivé nitro de formule générale (II.1.2) intermédiairement obtenu est ensuite réduit, par exemple, par le Nickel de Raney en présence d'hydrate d'hydrazine pour conduire aux anilines de formule générale (II.1).

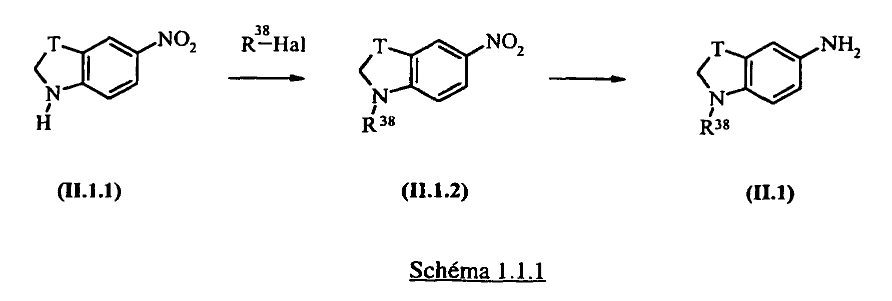

Par ailleurs, certains dérivés des phénylènediamines de formule générale (II.1), non commerciaux, peuvent être préparés selon Farmaco (1951) 6, 713-717.

Dans le cas particulier où A est un dérivé phénolique (A=A2), les anilines de formule générale (II.1) sont obtenues par hydrogénation, en présence de Pd/C, des dérivés nitrophénols précurseurs. Les dérivés nitrés des di-alkyl phénols sont accessibles selon les méthodes décrites dans J. Org. Chem. (1968) 33 (1), 223-226 ou J. Med. Chem. (1998), 41, 1846-1854.

Les intermédiaires de formule générale (II.1) dans lesquels A'1 est une diphénylamine, sont accessibles à partir des méthodes décrites dans la littérature (Synthesis (1990) 430 ; Indian J. Chem. (1981) 20B, 611-613 ; J. Med. Chem. (1975) 18 (4), 386-391) qui passent par la réduction d'un intermédiaire nitrodiphénylamine. La réduction de la fonction nitro est effectuée classiquement par hydrogénation en présence d'une quantité catalytique de Pd/C pour accéder aux aminodiphénylamines de formule générale (II.1).

Lorsque A est un dérivé carbazole (W représente alors une liaison directe), les méthodes de préparation des aminocarbazoles de formule générale (II.1) passent par la synthèse d'un intermédiaire nitrocarbazole. Ces méthodes sont décrites dans Pharmazie (1993) 48 (11), 817-820 ; Synth. Commun. (1994) 24(1), 1-10 ; J. Org. Chem. (1980) 45, 1493-1496 ; J. Org. Chem. (1964) 29 (8), 2474-2476 ; Org. Prep. Proced. Int. (1981) 13 (6), 419-421 ou J. Org. Chem. (1963) 28, 884. La réduction de la fonction nitro des intermédiaires nitrocarbazoles est, dans ce cas, effectuée de préférence à l'aide d'hydrate d'hydrazine en présence de Nickel de Raney.

Les intermédiaires de formule générale (II.1) dans lesquels A est un dérivé phénothiazine (W représente un atome de soufre), sont accessibles à partir de méthodes de la littérature qui passent par la synthèse d'un dérivé nitrophénothiazine. En particulier la 3-nitrophénothiazine est décrite dans J. Org. Chem. (1972) 37, 2691. La réduction de la fonction nitro pour accéder aux aminophénothiazines de formule générale (II.1) est effectuée classiquement par hydrogénation en présence d'une quantité catalytique de Pd/C dans un solvant tel que l'éthanol.

### 1.1.2) Préparation des intermédiaires (II.4) :

Les acide-esters de formule générale (II.4), schéma 1.1.2, peuvent être préparés à partir des diesters commerciaux de formule générale (II.4.1) selon une méthode décrite dans la littérature (Tetrahedron Asymmetry (1997) 8 (11), 1821-1823).

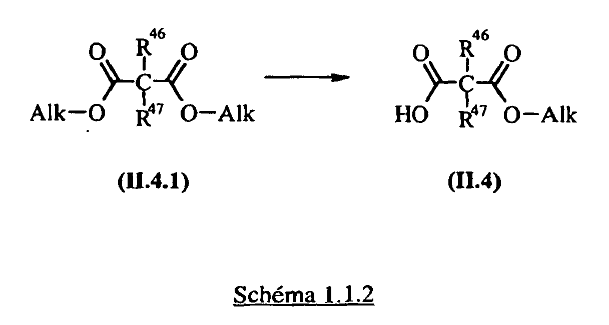

### 1.2) A partir de A-CO₂H :

Les intermédiaires acides carboxyliques de formule générale (II) sont également accessibles à partir de la condensation des acides carboxyliques de formule générale (II.8) avec les amino-esters commerciaux de formule générale (II.9A) ou (II.9B), schéma 1.2, au cours d'une étape de synthèse peptidique précédemment décrite. Les carboxamides intermédiairement obtenus (II.10A) et (II.10B) sont ensuite saponifiés pour conduire aux acides carboxyliques de formule générale (II).

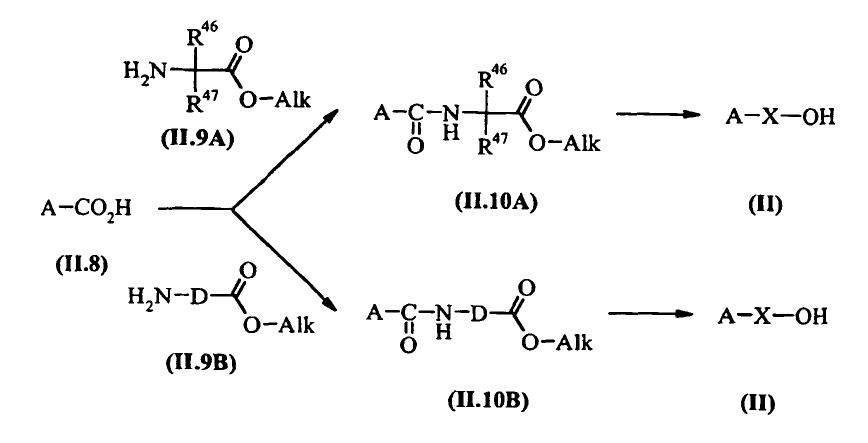

### 1.2.1) Préparation des intermédiaires (II.8) :

Les dérivés carboxyliques de formule générale (II.8), non accessibles commercialement, peuvent être préparés à partir de la littérature (p. ex. : J. Org. Chem. (1961) 26, 1221-1223 ; Acta Chem. Scandinavica (1973) 27, 888-890 ; Can. J. Chem. (1972) 50, 1276-1282 ; J. Med. Chem. (1992) 35(4), 716-724 ; J. Org. Chem. (1989) 54, 560-569 ; J. Med. Chem. (1998) 41(2), 148-156 ; Bull. Soc. Chim. Fr. (1960), 1049-1066)).

### 1.3) A partir de A-OH ou A-SH :

Les acides de formule générale (II) (schéma 1.3) dans lesquels X représente -O-(CH₂)ₙ₋CO-, sont préparés à partir des hydroquinones de formule générale (II.11) obtenues selon la littérature (J. Chem. Soc. Perkin 1 (1981) 303-306). La condensation sur des halogènoesters commerciaux de formule générale (II.12) est effectuée en présence d'une base telle que, par exemple K₂CO₃, en chauffant dans un solvant polaire comme, par exemple, le THF pendant au moins 5 heures. Les esters de formule générale (II.13) intermédiairement obtenus sont ensuite déprotégés (en milieu acide dans le cas des esters de tert-butyle) pour conduire aux acides de formule générale (II).

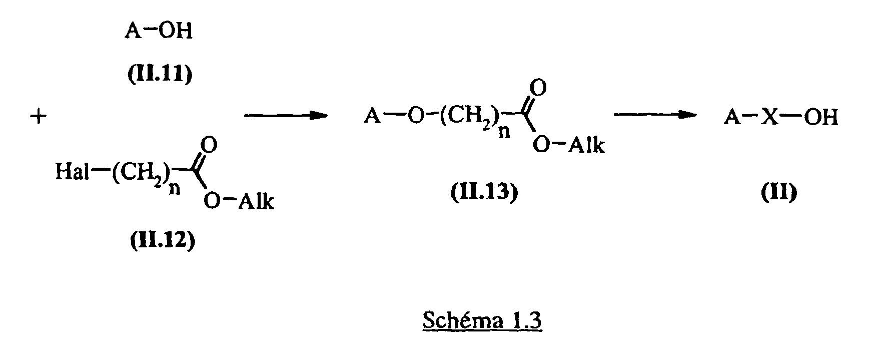

Les acides de formule générale (II) dans lesquels X représente -S-(CH₂)ₙ-CO-, sont préparés selon une méthode de la littérature (J. Med. Chem. (1997) 40 (12), 1906-1918).

### 1.4) A partir de A-CO₂H, lorsque Z représente un hétérocycle avec V = S ou O :

1.4.a) Dans les cas où Z représente un hétérocyle insaturé, les acides carboxyliques de formule générale (II), schéma 1.4a, peuvent être préparés à partir des acides carboxyliques de formule générale (II.8). 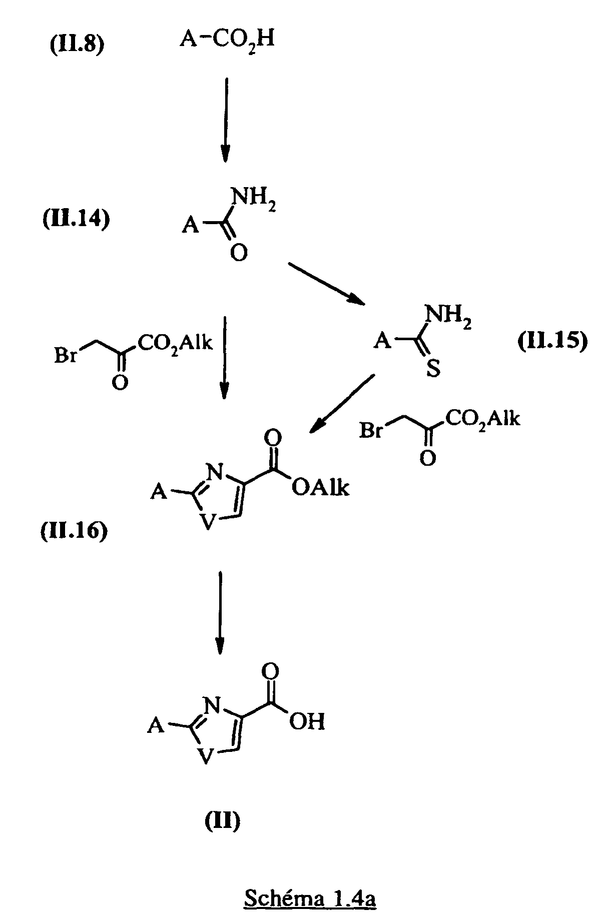
   La formation du carboxamide I^{aire} de formule générale (II.14) est effectuée selon un protocole expérimental décrit dans la littérature (Synthesis (1989), 1, 37). Par chauffage, entre 50° C et le reflux du solvant, pendant un temps compris entre une et 15 heures, de l'intermédiaire (II.14) en présence d'un bromopyruvate d'alkyle, on obtient les oxazoles (V = O) de formule générale (II.16). Alternativement, les thiazoles (V = S) de formule générale (II.16), sont accessibles en deux étapes à partir des carboxamides de formule générale (II.14). Ceux-ci en présence du réactif de Lawesson dans un solvant tel que, par exemple, le 1,4-dioxanne, conduisent classiquement aux thiocarboxamides de formule générale (II.15). L'étape de cyclisation est ensuite effectuée en présence de bromopyruvate d'alkyle comme précédemment décrit. Les acides carboxyliques de formule générale (II) sont finalement obtenus par déprotection de la fonction acide dans des conditions classiques.
1.4.b) Dans les cas où Z représente un hétérocycle saturé, et en particulier une thiazolidine, les acides carboxyliques de formule générale (II), schéma 1.4b, sont également accessibles à partir des acides carboxyliques de formule générale (II.8).

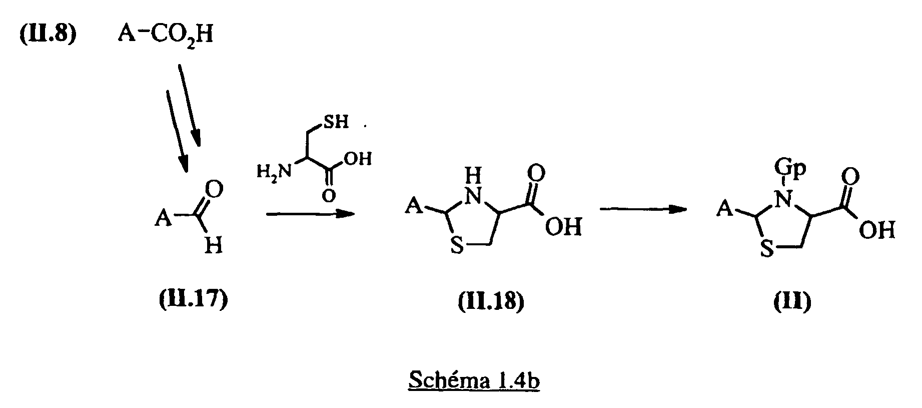

La préparation des aldéhydes de formule générale (II.17) est classiquement effectuée après activation de la fonction acide des intermédiaires de formule générale (II.8) sous forme d'ester ou d'alkylhydroxamate, en présence de DIBAL ou de LiAlH₄, selon des protocoles expérimentaux de la littérature (p. ex. J. Med. Chem. (1990) 33, 11-13). La réaction de ces aldéhydes avec la cystéine en présence de sels d'acétate conduit directement aux thiazolidines de formule générale (II.18) selon un protocole expérimental décrit dans J. Org. Chem. (1957) 22, 943-946. L'amine du cycle thiazolidine est ensuite protégée sous forme de carbamate (p.ex. Boc) dans les conditions classiques de la littérature pour conduire aux acides carboxyliques de formule générale (II).

### 1.5) A partir de A-N(R⁴⁵)-CO- :

Les acides carboxyliques de formule générale (II), dans lesquels X = -N(R⁴⁵)-(CH₂)ₙ-CO- avec n = 0, sont constitués d'une chaîne fonctionnalisée par une urée, schéma 1.5.

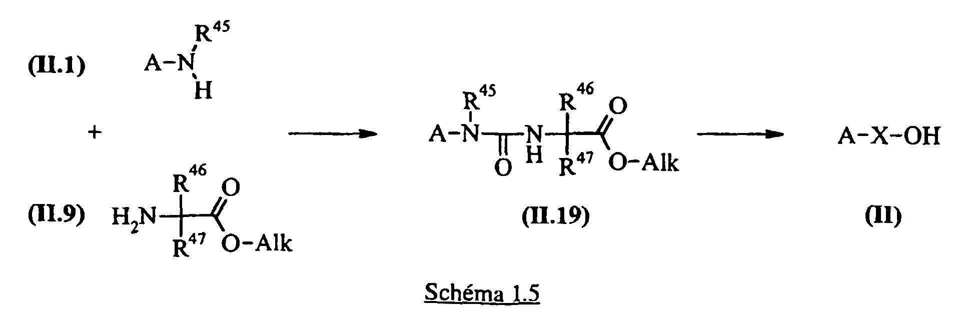

La synthèse de ces urées se fait par condensation des amines de formule générale (II.1) avec les aminoesters de formule générale (II.9) en présence de triphosgène et d'une amine tertiaire selon un protocole expérimental décrit dans la littérature (J. Org. Chem. (1994), 59(7), 1937-1938) pour conduire aux intermédiaires de formule générale (II.19). L'acide carboxylique de formule générale (II) est ensuite classiquement obtenu par déprotection de l'ester intermédiaire.

### 2) Synthèse des intermédiaires (III) :

La préparation des intermédiaires de formule générale (III), schéma 1.4, dans lesquels R² est tel que défini ci-dessus et Y = -(CH₂)ₚ-, avec p = 0, est effectuée à partir des dérivés de l'acide N-Cbz aspartique de formule générale (III.1) dont l'accès est décrit dans la littérature (J. Med. Chem. (1973) 16 (11), 1277-1280). Par chauffage de ces intermédiaires en présence de trioxanne et d'une quantité catalytique d'APTS au reflux d'un solvant tel que, par exemple, le toluène, (Synthesis (1989) 7, 542-544) on obtient les dérivés d'oxazolidinone de formule générale (III.2). La réduction de la fonction acide est alors effectuée à l'aide de B₂H₆.THF dans le THF telle que décrite dans Chem. Pharm. Bull. (1995) 43 (10), 1683-1691 et conduit aux alcools de formule générale (III.3). Ceux-ci sont ensuite traités en milieu basique, et l'intermédiaire (III.4) ainsi généré est cyclisé à l'aide d'un agent de déshydratation classique tel que, par exemple, le Dicyclohexylcarbodiimide pour obtenir la lactone substituée de formule générale (III.5). L'intermédiaire de formule générale (III) est obtenu après coupure du carbamate de benzyle à l'aide de Pd/C sous atmosphère d'hydrogène.

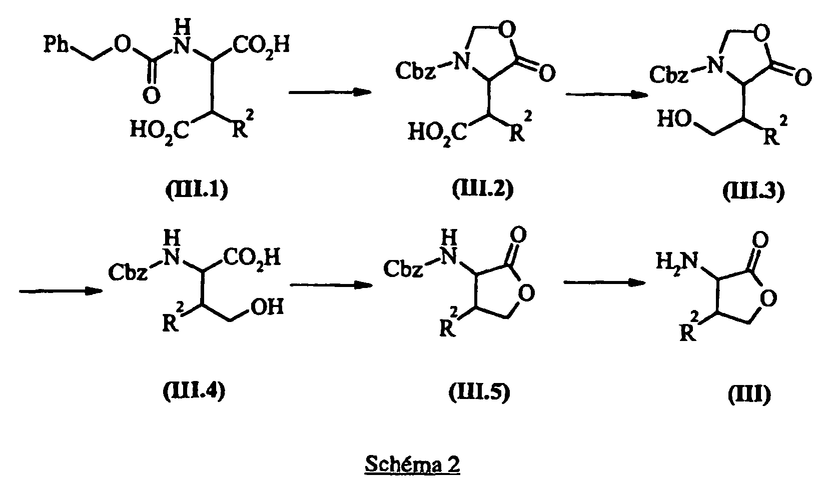

L'invention a également pour objet, à titre de produits industriels nouveaux, et notamment à titre de produits industriels nouveaux destinés à la préparation de produits de formule I, les produits répondant à l'une des formule suivantes :
N-1-(4-anilinophényl)-N-4-[(3S)-2-oxotétrahydro-3-furanyl]succinamide ;
(2S)-2-{[4-(4-anilinoanilino)-4-oxobutanoyl]amino}-4-méthylpentanoate de méthyle ;
N1-(4-anilinophényl)-N4-[(1S)-1-formyl-3-méthylbutyl]succinamide ;
benzyl 3-(4-anilinoanilino)-3-oxo-2-phénylpropanoate ;
acide 3-(4-anilinoanilino)-3-oxo-2-phénylpropanoïque ;
N-1-(4-anilinophényl)-N-3-[(3S)-2-oxotétrahydro-3-furanyl]-2-phénylmalonamide
3-(4-anilinoanilino)dihydro-2(3H)-furanone ;
(2S)-4-méthyl-2-[(10H-phénothiazin-2-ylcarbonyl)amino]pentanoate de méthyle ;
acide (2S)-4-méthyl-2-[(10H-phénothiazin-2-ylcarbonyl)amino]pentanoïque ;
N-[(1S)-3-méthyl-1-({[(3S)-2-oxotétrahydro-3-furanyl]amino}carbonyl)butyl]-10H-phénothiazine-2-carboxamide ;
2-(10H-phénothiazin-2-yl)-1,3-thiazole-4-carboxylate d'éthyle ;
acide 2-(10H-phénothiazin-2-yl)-1,3-thiazole-4-carboxylique ;
N-[(3S)-2-oxotétrahydro-3-furanyl]-2-(10H-phénothiazin-2-yl)-1,3-thiazole-4-carboxamide ;
4-[(10H-phenothiazin-2-ylcarbonyl)amino]benzoate de méthyle ;
acide 4-[(10H-phénothiazin-2-ylcarbonyl)amino]benzoïque ;
N-[4-({[(3S)-2-oxotétrahydro-3-furanyl]amino}carbonyl)phényl]-10H-phénothiazine-2-carboxamide ;
(2S)-4-méthyl-2-[(10H-phénothiazin-1-ylcarbonyl)amino]pentanoate de méthyle ;
acide (2S)-4-méthyl-2-[(10H-phénothiazin-1-ylcarbonyl)amino]pentanoïque ;
N-[(1S)-1-({[(3S)-2-oxotétrahydro-3-furanyl]amino}carbonyl)-3-méthylbutyl]-10H-phénothiazine-1-carboxamide ;
*N*-[(1*S*)-1-(1,4-dioxa-7-azaspiro[4.4]non-7-ylcarbonyl)-3-méthylbutyl]-10*H-*phénothiazine-2-carboxamide ;
2-(3,5-di-*tert*-butyl-4-hydroxyphénoxy)-*N*-[(3*S*)-2-oxotétrahydro-3-furanyl] acétamide
5-nitro-1-propylindoline ;
1-propyl-2,3-dihydro-1*H*-indol-5-ylamine;
3-oxo-2-phényl-N-(1-propyl-2,3-dihydro-1H-indol-5-yl)-béta-alanine ;
N¹-[(3S)-2-oxotétrahydro-3-furanyl]-2-phényl-N³-(1-propyl-2,3-dihydro-1H-indol-5-yl)malonamide;
*N*-(2-anilinophényl)-*N*'-[(3*S*)-2-oxotétrahydro-3-furanyl]urée ;
oxo[(1-propyl-2,3-dihydro-1H-indol-5-yl)amino]acétate d'éthyle ;
acide oxo[(1-propyl-2,3-dihydro-1H-indol-5-yl)amino]acétique ;
(2S)-2-({[(2R)-6-hydroxy-2,5,7,8-tétramethyl-3,4-dihydro-2H-chromen-2-yl]carbonyl}amino)-3-phenylpropanoate de méthyle ;
(2R)-N-[(1S)-1-benzyl-2-oxoéthyl]-6-hydroxy-2,5,7,8-tétramethyl-3,4-dihydro-2H-chromène-2-carboxamide ;
5-methyl 1,5-indolinedicarboxylate de *tert*-butyle ;
acide 1-(*tert*-butoxycarbonyl)-5-indolinecarboxylique ;
5-({[(3S)-2-oxotétrahydro-3-furanyl]amino}carbonyl)-1-indolinecarboxylate de *tert-*butyle ;
5-({[(3S)-2-hydroxytétrahydro-3-furanyl]amino}carbonyl)-1-indolinecarboxylate de *tert*-butyle.

### Partie expérimentale

Les exemples suivants sont présentés pour illustrer les procédures ci-dessus et ne doivent en aucun cas être considérés comme une limite à la portée de l'invention.

### Exemple 17: N-[(1S)-1-({[(3S)-2-hydroxytétrahydro-3-furanyl]amino}carbonyl)-3-méthylbutyl]-10H-phénothiazine-2-carboxamide :

### 17.1) (2S)-4-méthyl-2-[(10H-phénothiazin-2-ylcarbonyl)amino]pentanoate de méthyle :

A une solution de 1,82 g (10 mmoles) du chlorhydrate de l'ester méthylique de la L-Leucine, 2,43 g (10 mmoles) de l'acide 10H-phénothiazine-2-carboxylique (J. Med. Chem.(1998) 41 (2), 148-156), 1,48 g (11 mmoles) de HOBT et 4,21 g (22 mmoles) de EDC dans 30 ml de DMF anhydre, on ajoute 4,6 ml (33 mmoles) de triéthylamine. Le mélange réactionnel est agité pendant 15 heures. Après évaporation du solvant sous vide, le résidu est partitionné entre 100 ml d'AcOEt et 50 ml d'une solution 1M d'HCl. La phase organique est décantée et lavée successivement par 50 ml H₂O, 50 ml d'une solution saturée de N_{A}HCO₃ et 50 ml de saumure. La solution organique est séchée sur sulfate de magnésium, filtrée et concentrée à sec sous vide. Le résidu d'évaporation est repris par Et₂O et filtré. Poudre jaune (71 %). Point de fusion : 160,5-161° C.

### 17.2) Acide (2S)-4-méthyl-2-[(10H-phénothiazin-2-ylcarbonyl)amino]pentanoïque :

A une solution de 1,85 g (5 mmoles) de l'intermédiaire 17.1 dans 20 ml de THF, on ajoute en une fois une solution de 0,44 g (11 mmoles) de LiOH, H₂O dans 20 ml d'H₂O. Le mélange réactionnel est agité 1 h 30 à 20° C. L'ensemble est refroidi à l'aide d'un bain de glace avant l'addition d'une solution aqueuse concentrée d'HCl jusqu'à pH acide. Après dilution par 100 ml d'AcOEt et agitation, la phase organique est décantée. Celle-ci est ensuite lavée par 20 ml d'une solution aqueuse 1M d'HCl suivi de 20 ml de saumure. La solution organique est séchée sur sulfate de sodium, filtrée et concentrée à sec sous vide. Poudre jaune-vert. Le produit est utilisé tel quel dans l'étape suivante.

### 17.3) N-[(1S)-3-méthyl-1-({[(3S)-2-oxotétrahydro-3-furanyl]amino}carbonyl)butyl]-10H-phénothiazine-2-carboxamide :

Le protocole expérimental utilisé est le même que celui décrit pour l'intermédiaire 12.2, l'intermédiaire 17.2 remplaçant l'intermédiaire 12.1. Poudre jaune. Point de fusion : 151-152° C.

### 17.4) N-[(1S)-1-({[(3S)-2-hydroxytétrahydro-3-furanyl]amino}carbonyl)-3-méthyl butyl]-10H-phénothiazine-2-carboxamide :

Le protocole expérimental utilisé est le même que celui décrit pour l'intermédiaire 1.2, l'intermédiaire 17.3 remplaçant l'intermédiaire 1.1. Poudre jaune. Point de fusion : 100-101° C

### Exemple 18 : Acétate de (3S)-3-({(2S)-4-méthyl-2-[(10H-phénothiazin-2-ylcarbonyl)-amino]pentanoyl}amino)tétrahydro-2-furanyl :

Le protocole expérimental utilisé est le même que celui décrit pour l'exemple 13, à partir de l'intermédiaire 17.4. Les deux diastéréoisomères 18.1 et 18.2 sont séparés par chromatographie sur colonne de silice (éluant : Heptane/AcOEt : 1/1).

### 18.1) Acétate de (2R,3S)-3-({(2S)-4-méthyl-2-[(10H-phénothiazin-2-ylcarbonyl)amino]-pentanoyl}amino)tétrahydro-2-furanyl :

Poudre jaune pâle. Point de fusion : 199-201° C.

### 18.2) Acétate de (2S,3S)-3-({(2S)-4-méthyl-2-[(10H-phénothiazin-2-ylcarbonyl) amino]-pentanoyl}amino)tétrahydro-2-furanyl :

Poudre jaune pâle. Point de fusion : 205-208° C.

### Exemple 19 : N-[(3S)-2-hydroxytétrahydro-3-furanyl]-2-(10H-phénothiazin-2-yl)-1,3-thiazole-4-carboxamide :

### 19.1) 10H-phénothiazine-2-carbothioamide :

Un mélange réactionnel composé de 3,4 g (14 mmoles) de 10*H*-phénothiazine-2-carboxamide (J. Org. Chem. (1961) 26, 1138-1143) et de 3,4 g (8,4 mmoles) de réactif de Lawesson en solution dans 40 ml de 1,4-dioxanne additionné de 20 ml de pyridine est chauffé à 110° C pendant 1 h 30. La solution brune est ensuite concentrée sous vide et le résidu est dilué dans 200 ml d'AcOEt et 100 ml d'H₂O. Après agitation et décantation, la phase organique est lavée successivement par 100 ml d'une solution aqueuse 1N d'HCl et 100 ml de saumure. Après séchage sur sulfate de sodium, filtration et évaporation du solvant sous vide on obtient une poudre orange. Cette poudre est lavée par Et₂O, le filtrat est éliminé, et extraite par de l'acétone. Le filtrat acétonique est alors concentré sous vide et le résidu d'évaporation est alors purifié sur une colonne de silice (éluant : Heptane/AcOEt : 1/1 jusqu'à 4/6). Poudre orange. Point de fusion : 208-209° C.

### 19.2) 2-(10H-phénothiazin-2-yl)-1,3-thiazole-4-carboxylate d'éthyle :

A une suspension de 1,43 g (5,53 mmoles) de l'intermédiaire 19.1 dans 70 ml d'EtOH absolu, on ajoute 2,09 ml (16,5 mmoles) de bromopyruvate d'éthyle. Le mélange réactionnel est alors chauffé pendant 1 h 30 à reflux. Après concentration à sec sous vide, le résidu noir obtenu est lavé par Et₂O avant d'être déposé au sommet d'une colonne de chromatographie (élution : Heptane/AcOEt/THF : 6/4/0 jusqu'à THF pur). Poudre jaune (83 %).

### 19.3) Acide 2-(10H-phénothiazin-2-yl)-1,3-thiazole-4-carboxylique :

Une solution de l'intermédiaire 19.2 (1,62 g, 4,57 mmoles) dans 50 ml de THF est refroidie à 0° C avant l'addition en une portion d'une solution de 300 mg (7,3 mmoles) de NaOH dans 30 ml H₂O. L'agitation est poursuivie 15 heures à 20° C avant d'acidifier le mélange réactionnel, à 0° C, par une solution aqueuse d'HCl concentrée. Le produit est alors extrait à l'aide de 100 ml d'AcOEt et la solution organique est lavée par 25 ml d'H₂O suivi de saumure. Après séchage sur sulfate de sodium, filtration et concentration sous vide, le résidu est purifié sur une colonne de silice (éluant : CH₂Cl₂/MeOH: 8/2 jusqu'à 1/1). Poudre jaune.

### 19.4) N-[(3S)-2-oxotétrahydro-3-furanyl]-2-(10H-phénothiazin-2-yl)-1,3-thiazole-4-carboxamide :

Le protocole expérimental utilisé est le même que celui décrit pour l'intermédiaire 12.2, l'intermédiaire 19.3 remplaçant l'intermédiaire 12.1. Poudre jaune. Point de fusion : 277-277,5° C.

### 19.5) N-[(3S)-2-hydroxytétrahydro-3-furanyl]-2-(10H-phénothiazin-2-yl)-1,3-thiazole-4-carboxamide :

Le protocole expérimental utilisé est le même que celui décrit pour l'intermédiaire 1.2, l'intermédiaire 19.4 remplaçant l'intermédiaire 1.1. Poudre jaune. Point de fusion : 189-190° C.

### Exemple 20 : N-[4-({[(3S)-2-hydroxytétrahydro-3-furanyl]amino}carbonyl)phényl]-10H-phénothiazine-2-carboxamide :

### 20.1) Acide 4-[(10H-phénothiazin-2-ylcarbonyl)amino]benzoïque :

Le protocole expérimental utilisé est le même que celui décrit pour les synthèses des intermédiaires 17.1 et 17.2, l'acide 4-aminobenzoate de méthyle remplaçant l'ester méthylique de la L-Leucine.

### 20.2) N-[4-({[(3S)-2-oxotétrahydro-3-furanyl]amino}carbonyl)phényl]-10H-phénothiazine-2-carboxamide :

Le protocole expérimental utilisé est le même que celui décrit pour la synthèse de l'intermédiaire 12.2, l'intermédiaire 20.1 remplaçant l'acide 4-(4-anilinoanilino)-4-oxobutanoïque. Poudre jaune-vert. Point de fusion : 284-285° C.

### 20.3) N-[4-({[(3S)-2-hydroxytétrahydro-3-furanyl]amino}carbonyl)phényl]-10H-phénothiazine-2-carboxamide :

Le protocole expérimental utilisé est le même que celui décrit pour l'intermédiaire 1.2, l'intermédiaire 20.2 remplaçant l'intermédiaire 1.1. Poudre jaune foncée. Point de fusion : 234-235° C.

### Exemple 21 : N-[(1S)-1-({[(3S)-2-hydroxytetrahydro-3-furanyl]amino}carbonyl)-3-methylbutyl]-10H-phenothiazine-1-carboxamide :

Le protocole expérimental utilisé est identique à celui décrit pour le composé 17, l'acide 10*H*-phénothiazine-1-carboxylique remplaçant l'acide 10*H*-phénothiazine-2-carboxylique. Poudre jaune. Point de fusion : 99-101° C.

### Exemple 22 : Pivalate de (3S)-3-({(2S)-4-méthyl-2-[(10H-phénothiazin-2-ylcarbonyl) amino]pentanoyl} amino)tétrahydro-2-furanyl :

On additionne goutte-à goutte 0,14 ml de chlorure de 2,2-dimethylpropanoyle à une solution de 0,45 g (1,02 mmole) de l'intermédiaire 17.4 et de 0,28 ml (2,04 mmoles) de Et₃N dans 20 ml de CH₂Cl₂ refroidie à 0°C. Le mélange réactionnel est ensuite agité 24 heures à 22°C. Après dilution par 50 ml de CH₂Cl₂, la solution organique est lavée par 20 ml d'eau suivi de 20 ml de saumure, séchée sur MgSO₄, filtrée et concentrée à sec sous vide. Le produit est finalement purifié par chromatographie sur une colonne de silice (éluant : Heptane/AcOEt : 1/1). Solide jaune pâle. Point de fusion : 107-109°C.

### Exemple 23 : 3,3-diméthylbutanoate de (3S)-3-({(2S)-4-méthyl-2-[(10H-phénothiazin-2-ylcarbonyl)amino]pentanoyl}amino)tétrahydro-2-furanyl :

Le protocole expérimental utilisé est le même que celui décrit pour l'exemple 22, à partir de l'intermédiaire 17.4 et du chlorure de 3,3-dimethylbutanoyle. Solide jaune. Point de fusion : 111-113°C.

### Exemple 24 : (3S)-3-({(2S)-4-méthyl-2-[(10H-phénothiazin-2-ylcarbonyl)amino] pentanoyl}amino)tétrahydro-2-furanyl benzoate :

Le protocole expérimental utilisé est le même que celui décrit pour l'exemple 22, à partir de l'intermédiaire 17.4 et du chlorure de benzoyle. Solide jaune pâle. Point de fusion : 193-195°C.

### Exemple 25 : (3S)-3-({(2S)-4-méthyl-2-[(10H-phénothiazin-2-ylcarbonyl)amino] pentanoyl}amino)tétrahydro-2-furanyl phénylacetate :

Le protocole expérimental utilisé est le même que celui décrit pour l'exemple 22, à partir de l'intermédiaire 17.4 et du chlorure de phénylacétyle. Solide jaune. LC-MS : MH⁺ = 560,2.

### Exemple 26 : (3S)-3-({(2S)-4-méthyl-2-[(10H-phénothiazin-2-ylcarbonyl)amino] pentanoyl}amino)tétrahydro-2-furanyl (2S)-2-(diméthylamino)-3-phénylpropanoate :

A une solution de 0,5 g (1,13 mmole) de l'intermédiaire 17.4 dans 2 ml de CH₂Cl₂, on ajoute 0,22 g (1,13 mmole) de l'acide (2S)-2-(diméthylamino)-3-phénylpropanoïque et 0,23 g (1,13 mmole) de 1,3-dicylohexylcarbodiimide. Après 72 h d'agitation à 22°C, le précipité est filtré et lavé par 10 ml de CH₂Cl₂. Le filtrat est ensuite lavé par une solution saturée de NaHCO₃ (10 ml) suivi de 10 ml d'eau et 10 ml de saumure. La solution organique est séchée sur MgSO₄, filtrée et concentrée à sec. Le résidu d'évaporation est purifié sur une colonne de silice (éluant : AcOEt). Solide jaune. LC-MS : MH⁺ = 617,2.

### Exemple 27 : 4-morpholinecarboxylate de (3S)-3-({(2S)-4-méthyl-2-[(10H-phénothiazin-2-ylcarbonyl)amino]pentanoyl} amino)tétrahydro-2-furanyl:

Le protocole expérimental utilisé est le même que celui décrit pour l'exemple 22, à partir de l'intermédiaire 17.4 et de chloroformiate de morpholine. Solide jaune. Point de fusion : 165-167°C.

### Etude pharmacologique des produits de l'invention

### Etude des effets sur la Calpaine I porcine :

La méthode utilisée est celle décrite par Mallya et coll. (Biochemical and biophysical research communications 248 293-296 (1998). L'activité calpain *in vitro* est suivie par la mesure de la fluorescence due à la dégradation d'un substrat artificiel de l'enzyme Suc-LY-AMC (Suc-Leu-Tyr-aminométhylcoumarine). L'aminométhylcoumarine libérée fluoresce à 460 nm sous une excitation à 380 nm. Les inhibiteurs testés sont dissous dans du DMSO à 40 fois la concentration finale. 5 *µ*l de solution sont déposés dans une plaque 96 puits à paroi noire. 175 *µ*l/puits de tampon de réaction contenant la calpain I et son substrat sont alors ajoutés. La réaction est démarrée par l'ajout de 20 *µ*l de CaCl₂ 50 mM. Les plaques sont incubées à 25° C et la fluorescence (380 nm excitation et 460 nm émission) est lue au bout de 30 minutes à l'aide d'un lecteur de microplaques (Victor, Wallack). Les CI₅₀ sont déterminées par le calcul des rapports fluorescence produit / fluorescence témoin DMSO. Composition du tampon de la réaction enzymatique : Tris-HCl 50 mM pH 7.5, NaCl 50 mM, EDTA 1 mM, EGTA 1 mM, b-Mercaptoéthanol 5 mM, Suc-LY-AMC 1mM (Bachem, ref. 1-1355) et 2.5 U/ml Calpain I (érythrocytes porcins, Calbiochem ref 208712).). Sur ce test, la valeur IC₅₀ de certains composés selon l'invention est inférieure à 5µM.

### Etude des effets sur la péroxidation lipidique du cortex cérébral de rat :

L'activité inhibitrice des produits de l'invention est déterminée par la mesure de leurs effets sur le degré de péroxidation lipidique, déterminée par la concentration en malondialdéhyde (MDA). Le MDA produit par la péroxidation des acides gras insaturés est un bon indice de la péroxidation lipidique (H Esterbauer and KH Cheeseman, *Meth. Enzymol.* (1990) 186 : 407-421). Des rats mâles Sprague Dawley de 200 à 250 g (Charles River) ont été sacrifiés par décapitation. Le cortex cérébral est prélevé, puis homogénéisé au potter de Thomas dans du tampon Tris-HCl 20 mM, pH = 7,4. L'homogénat est centrifugé deux fois à 50 000 g pendant 10 minutes à 4° C. Le culot est conservé à -80° C. Le jour de l'expérience, le culot est remis en suspension à la concentration de 1 g /15 ml et centrifugé à 515 g pendant 10 minutes à 4°C. Le surnageant est utilisé immédiatement pour la détermination de la péroxidation lipidique. L'homogénat de cortex cérébral de rat (500 µl) est incubé à 37° C pendant 15 minutes en présence des composés à tester ou du solvant (10 µl). La réaction de péroxidation lipidique est initiée par l'ajout de 50 µl de FeCl₂ à 1 mM, d'EDTA à 1 mM et d'acide ascorbique à 4 mM. Après 30 minutes d'incubation à 37° C, la réaction est arrêtée par l'ajout de 50 µl d'une solution de di tertio butyl toluène hydroxylé (BHT, 0,2 %). Le MDA est quantifié à l'aide d'un test colorimétrique, en faisant réagir un réactif chromogène (R) le N-méthyl-2-phénylindole (650 µl) avec 200 µl de l'homogénat pendant 1 heure à 45° C. La condensation d'une molécule de MDA avec deux molécules de réactif R produit un chromophore stable dont la longueur d'onde d'absorbance maximale est égale à 586 nm. (Caldwell et coll. European J. Pharmacol. (1995) 285, 203-206). Sur ce test, la valeur IC₅₀ des composés selon l'invention est inférieure à 5 *µ*M.

## Revendications

1. Composés de formule générale (I) 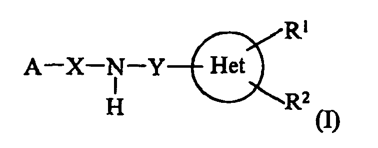
sous forme racémique, d'énantiomères, de diastéréoisomères ou toutes combinaisons de ces formes, dans laquelle
R¹ représente un atome d'hydrogène, un radical -OR³, -SR³, oxo ou un acétal cyclique,
dans lequel R³ représente un atome d'hydrogène, un radical alkyle, arylalkyle, hétérocycloalkylcarbonyle, alkylcarbonyle, arylcarbonyle ou aralkylcarbonyle,
dans lesquels les radicaux alkyle, aryle ou hétérocycloalkyle sont éventuellement substitués par un ou plusieurs substituants identiques ou différents choisis parmi : alkyle, OH, alkoxy, nitro, cyano, halogène ou -NR⁴R⁵ ;
R⁴ et R⁵ représentent, indépendamment, un atome d'hydrogène ou un radical alkyle, ou bien R⁴ et R⁵ forment ensemble avec l'atome d'azote auquel ils sont rattachés un hétérocycle éventuellement susbtitué,
R² représente un atome d'hydrogène, un radical alkyle, aryle ou aralkyle, le groupement aryle étant éventuellement substitué par un ou plusieurs radicaux identiques ou différents choisis parmi : -OR⁶, -NR⁷R⁸, halogène, cyano, nitro ou alkyle,
dans lequel R⁶, R⁷ et R⁸ représentent, indépendamment, un atome d'hydrogène, un radical alkyle, aryle, aralkyle, alkylcarbonyle, arylcarbonyle ou aralkylcarbonyle ;
A représente un radical A1 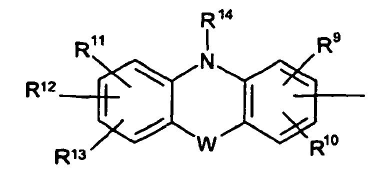
dans lequel R⁹, R¹⁰, R¹¹, R¹², R¹³ représentent, indépendamment, un atome d'hydrogène, un halogène, le groupe OH, un radical alkyle, alkoxy, cyano, nitro ou -NR¹⁵R¹⁶,
R¹⁵ et R¹⁶ représentent, indépendamment, un atome d'hydrogène, un radical alkyle ou un groupe -COR¹⁷, ou bien R¹⁵ et R¹⁶ forment ensemble avec l'atome d'azote auquel ils sont rattachés un hétérocycle éventuellement substitué,
R¹⁷ représente un atome d'hydrogène, un radical alkyle, alkoxy ou -NR¹⁸R¹⁹,
R¹⁸ et R¹⁹ représentent, indépendamment, un atome d'hydrogène ou un radical alkyle, ou bien R¹⁸ et R¹⁹ forment ensemble avec l'atome d'azote auquel ils sont rattachés un hétérocycle éventuellement substitué,
R¹⁴ représente un atome d'hydrogène, un radical alkyle ou un groupe -COR²⁰,
R²⁰ représente un atome d'hydrogène, un radical alkyle, alkoxy, aryle, aralkyle, hétérocycloalkyle ou -NR²¹R²²,
dans lesquels les radicaux alkyle, aryle ou hétérocycloalkyle sont éventuellement substitués par un ou plusieurs substituants identiques ou différents choisis parmi : alkyle, OH, alkoxy, nitro, cyano, halogène ou NR⁴R⁵;
R²¹ et R²² représentent, indépendamment, un atome d'hydrogène ou un radical alkyle, ou bien R²¹ et R²² forment ensemble avec l'atome d'azote auquel ils sont rattachés un hétérocycle éventuellement substitué,
W représente une liaison, O ou S ou encore un radical -NR²³, dans lequel R²³ représente un atome d'hydrogène ou un radical alkyle ;
X représente -(CH₂)ₙ , -(CH₂)ₙ CO-, -N(R⁴⁵)-CO-(CH₂)ₙCO-, -N(R⁴⁵)-CO-D-CO-, -CO-N(R⁴⁵)-D-CO-, -CO-D-CO-, -CH=CH-(CH₂)ₙ-CO-, -N(R⁴⁵)-(CH₂)ₙ-CO-, -N(R⁴⁵)-CO-C(R⁴⁶R⁴⁷)-CO-, -O-(CH₂)ₙ-CO-, -N(R⁴⁵)-CO-NH-C(R⁴⁶R⁴⁷)-CO-, -CO-N(R⁴⁵)-C(R⁴⁶R⁴⁷)-CO-, -S-(CH₂)ₙ-CO- ou -Z-CO- ;
D représente un radical phénylène éventuellement substitué par un ou plusieurs radicaux identiques ou différents choisis parmi alkyle, alkoxy, OH, nitro, halogène, cyano, ou carboxyl éventuellement estérifié par un radical alkyle ;
Z représente un hétérocycle,
R⁴⁵ représente un atome d'hydrogène ou un radical alkyle,
R⁴⁶ et R⁴⁷ représentent, indépendamment, un atome d'hydrogène, un radical alkyle, aryle ou aralkyle dont les groupements alkyle et aryle sont éventuellement substitués par un ou plusieurs substituants identiques ou différents choisis parmi : le groupe OH, -SH, halogène, nitro, alkyle, alkoxy, alkylthio, aralkoxy, aryl-alkylthio, -NR⁴⁸R⁴⁹ et carboxyl éventuellement estérifié par un radical alkyle ;
R⁴⁸ et R⁴⁹ représentent, indépendamment, un atome d'hydrogène, un radical alkyle ou un groupe -COR⁵⁰, ou bien R⁴⁸ et R⁴⁹ forment ensemble avec l'atome d'azote auquel ils sont rattachés un hétérocycle éventuellement substitué,
R⁵⁰ représente un atome d'hydrogène, un radical alkyle, alkoxy ou -NR⁵¹R⁵²,
R⁵¹ et R⁵² représentent, indépendamment, un atome d'hydrogène ou un radical alkyle, ou bien R⁵¹ et R⁵² forment ensemble avec l'atome d'azote auquel ils sont rattachés, un hétérocycle éventuellement substitué ;
n étant un entier compris entre 0 et 6 ;
Y représente -(CH₂)ₚ-, -C(R⁵³R⁵⁴)-(CH₂)ₚ-, -C(R⁵³R⁵⁴)-CO-;
R⁵³ et R⁵⁴ représentent, indépendamment, un atome d'hydrogène, un radical alkyle, un radical aralkyle dont le groupement aryle est éventuellement substitué par un ou plusieurs substituants identiques ou différents choisis parmi : le groupe OH, halogène, nitro, alkyle, alkoxy, -NR⁵⁵R⁵⁶,
R⁵⁵ et R⁵⁶ représentent, indépendamment, un atome d'hydrogène, un radical alkyle ou un groupe -COR⁵⁷, ou bien R⁵⁵ et R⁵⁶ forment ensemble avec l'atome d'azote auquel ils sont rattachés, un hétérocycle éventuellement substitué,
R⁵⁷ représente un atome d'hydrogène, un radical alkyle, alkoxy ou -NR⁵⁸R⁵⁹,
R⁵⁸ et R⁵⁹ représentent, indépendamment, un atome d'hydrogène ou un radical alkyle, ou bien R⁵⁸ et R⁵⁹ forment ensemble avec l'atome d'azote auquel ils sont rattachés un hétérocycle éventuellement substitué ;
p étant un entier compris entre 0 et 6 ;
Het représente le radical tétrahydrofuran-3-yl,
ainsi que les sels d'addition avec les acides minéraux et organiques ou avec les bases minérales et organiques desdits composés de formule générale (I),
à l'exclusion des composés de formule (I) dans laquelle lorsque Het représente tétrahydrofuranne ou tétrahydropyranne, R¹ le radical OR³ avec R³ représentant un atome d'hydrogène, un radical alkyle, arylalkyle, hétérocycloalkylcarbonyle dont le radical hétérocycloalkyle est branché par un atome de carbone, alkylcarbonyle, arylcarbonyle ou aralkylcarbonyle, R² un hydrogène et Y le radical -(CH₂)ₚ- avec p = 0, alors X représente -CO-N(R⁴⁵)-C(R⁴⁶R⁴⁷)-CO- avec R⁴⁵ = R⁴⁶ = H.

2. Composés selon la revendication 1, **caractérisés en ce que** R¹ représente l'atome d'hydrogène, le radical -OR³ ou oxo.

3. Composés selon l'une des revendications précédentes, **caractérisés en ce** X représente -(CH₂)ₙ-, -(CH₂)ₙ-CO-, -O-(CH₂)ₙ-CO-, -CO-N(R⁴⁵)-D-CO-, -N(R⁴⁵)-CO-(CH₂)ₙ-CO-, -N(R⁴⁵)-CO-C(R⁴⁶R⁴⁷)-CO-, -N(R⁴⁵)-CO-NH-C(R⁴⁶R⁴⁷)-CO-, -N(R⁴⁵)-(CH₂)ₙ-CO-, -CO-N(R⁴⁵)-C(R⁴⁶R⁴⁷)-CO ou -Z-CO-.

4. Composés selon la revendication 3, **caractérisés en ce que** R⁴⁵ et R⁴⁷ représentent l'atome d'hydrogène, R⁴⁶ l'atome d'hydrogène, un radical alkyle ou phényle, D le radical phénylène et Z le radical thiazole.

5. Composés selon l'une des revendications précédentes, **caractérisés en ce que** R² représente un atome d'hydrogène ou un radical aralkyle, et de préférence benzyle.

6. Composés selon l'une des revendications précédentes, **caractérisés en ce que** A représente A1 avec W représentant l'atome de soufre.

7. Composés selon l'une des revendications précédentes, **caractérisés en ce que** A représente le radical

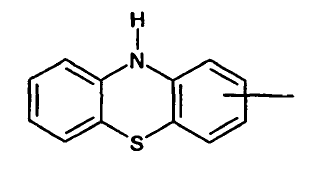

8. Composés répondant à l'une des formules suivantes :
N-[(1S)-1-({[(3S)-2-hydroxytétrahydro-3-furanyl]amino}carbonyl)-3-méthylbutyl]-10*H-*phénothiazine-2-carboxamide ;
Acétate de (3*S*)-3-({(2*S*)-4-méthyl-2-[(10*H*-phénothiazin-2-ylcarbonyl)-amino]pentanoyl} amino)tétrahydro-2-furanyl ;
N-[(3S)-2-hydroxytétrahydro-3-furanyl]-2-(10*H*-phénothiazin-2-yl)-1,3-thiazole-4-carboxamide ;
N-[4-({[(3S)-2-hydroxytétrahydro-3-furanyl]amino}carbonyl)phényl]-10*H-*phénothiazine-2-carboxamide ;
N-[(1S)-1-({[(3S)-2-hydroxytétrahydro-3-furanyl]amino}carbonyl)-3-méthylbutyl]-10*H*-phénothiazine-1-carboxamide ;
Pivalate de (3S)-3-({(2S)-4-méthyl-2-[(10*H*-phénothiazin-2-ylcarbonyl)amino] pentanoyl}amino)tétrahydro-2-furanyl ;
3,3-diméthylbutanoate de (3S)-3-({(2S)-4-méthyl-2-[(10*H*-phénothiazin-2-ylcarbonyl) amino]pentanoyl}amino)tétrahydro-2-furanyl ;
(3S)-3-({(2S)-4-méthyl-2-[(10*H*-phénothiazin-2-ylcarbonyl)amino]pentanoyl}amino) tétrahydro-2-furanyl benzoate ;
(3S)-3-({(2S)-4-méthyl-2-[(10*H*-phénothiazin-2-ylcarbonyl)amino]pentanoyl}amino) tétrahydro-2-furanyl phénylacetate ;
(3S)-3-({(2S)-4-méthyl-2-[(10*H*-phénothiazin-2-ylcarbonyl)amino]pentanoyl}amino) tétrahydro-2-furanyl (2*S*)-2-(diméthylamino)-3-phénylpropanoate ;
4-morpholinecarboxylate de (3S)-3-({(2S)-4-méthyl-2-[(10*H*-phénothiazin-2-ylcarbonyl) amino]pentanoyl}amino)tétrahydro-2-furanyl ;

9. Composés répondant à l'une des formules suivantes :
N-[(1S)-1-{{[(3S)-2-hydroxytétrahydro-3-furanyl]amino}carbonyl)-3-méthylbutyl]-10H-phénothiazine-2-carboxamide ;
Acétate de (3*S*)-3-({(2*S*)-4-méthyl-2-[(10*H*-phénothiazin-2-ylcarbonyl)-amino] pentanoyl}amino)tétrahydro-2-furanyl ;
Acétate de (2R,3S)-3-({(2S)-4-méthyl-2-[(10H-phénothiazin-2-ylcarbonyl)amino]-pentanoyl}amino)tétrahydro-2-fumyl ;
Acétate de (2S,3S)-3-({(2S)-4-méthyl-2-[(10H-phénothiazin-2-ylcarbonyl) amino]-pentanoyl}amino)tétrahydro-2-furanyl ; ainsi que les sels d'addition avec les acides minéraux et organiques ou avec les bases minérales et organiques desdits composés.

10. A titre de médicaments, des composés selon l'une des revendications 1 à 9.

11. Compositions pharmaceutiques comprenant, à titre de principe actif, au moins un médicament tel que défini à la revendication 10.

12. Utilisation de composés de formule (Iₐ) telle que définie ci-dessus, 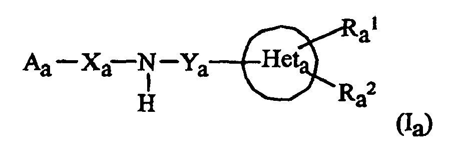
sous forme racémique, d'énantiomères, de diastéréoisomères ou toutes combinaisons de ces formes, dans laquelle
Rₐ¹ représente un atome d'hydrogène, un radical -OR³, -SR³, oxo ou un acétal cyclique,
dans lequel R³ représente un atome d'hydrogène, un radical alkyle, arylalkyle, hétérocycloalkylcarbonyle, alkylcarbonyle, arylcarbonyle ou aralkylcarbonyle,
dans lesquels les radicaux alkyle, aryle ou hétérocycloalkyle sont éventuellement substitués par un ou plusieurs substituants identiques ou différents choisis parmi : alkyle, OH, alkoxy, nitro, cyano, halogène ou -NR⁴R⁵ ;
R⁴ et R⁵ représentent, indépendamment, un atome d'hydrogène ou un radical alkyle, ou bien R⁴ et R⁵ forment ensemble avec l'atome d'azote auquel ils sont rattachés un hétérocycle éventuellement susbtitué,
Rₐ² représente un atome d'hydrogène, un radical alkyle, aryle ou aralkyle, le groupement aryle étant éventuellement substitué par un ou plusieurs radicaux identiques ou différents choisis parmi : -OR⁶, NR⁷R⁸, halogène, cyano, nitro ou alkyle,
dans lequel R⁶, R⁷ et R⁸ représentent, indépendamment, un atome d'hydrogène, un radical alkyle, aryle, aralkyle, alkylcarbonyle, arylcarbonyle ou analkylcarbonyle;
Aₐ représente un radical A1 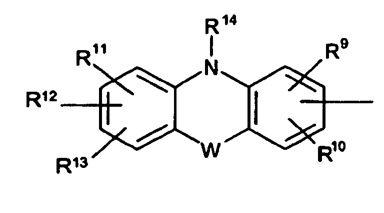
dans lequel R⁹, R¹⁰, R¹¹, R¹², R¹³ représentent, indépendamment, un atome d'hydrogène, un halogène, le groupe OH, un radical alkyle, alkoxy, cyano, nitro ou -NR¹⁵SR¹⁶,
R¹⁵ et R¹⁶ représentent, indépendamment, un atome d'hydrogène, un radical alkyle ou un groupe -COR¹⁷, ou bien R¹⁵ et R¹⁶ forment ensemble avec l'atome d'azote auquel ils sont rattachés un hétérocycle éventuellement substitué,
R¹⁷ représente un atome d'hydrogène, un radical alkyle, alkoxy ou -NR¹⁸R¹⁹,
R¹⁸ et R¹⁹ représentent, indépendamment, un atome d'hydrogène ou un radical alkyle, ou bien R¹⁸ et R¹⁹ forment ensemble avec l'atome d'azote auquel ils sont rattachés un hétérocycle éventuellement substitué,
R¹⁴ représente un atome d'hydrogène, un radical alkyle ou un groupe -COR²⁰,
R²⁰ représente un atome d'hydrogène, un radical alkyle, alkoxy, aryle, aralkyle, hétérocycloalkyle ou -NR²¹R²²,
dans lesquels les radicaux alkyle, aryle ou hétérocycloallcyle sont éventuellement substitués par un ou plusieurs substituants identiques ou différents choisis parmi : alkyle, OH, alkoxy, nitro, cyano, halogène ou -NR⁴R⁵ ;
R²¹ et R²² représentent, indépendamment, un atome d'hydrogène ou un radical alkyle, ou bien R²¹ et R²² forment ensemble avec l'atome d'azote auquel ils sont rattachés un hétérocycle éventuellement substitué,
W représente une liaison, O ou S ou encore un radical -NR²³, dans lequel R²³ représente un atome d'hydrogène ou un radical alkyle ;
Xₐ représente -(CH₂)ₙ-, -(CH₂)ₙ-CO-, -N(R⁴⁵)-CO-(CH₂)ₙ-CO-, -N(R⁴⁵)-CO-D-CO-, -CO-N(R⁴⁵)-D-CO-, -CO-D-CO-, -CH=CH-(CH₂)ₙ-CO-, -N(R⁴⁵)-(CH₂)ₙ-CO-, -N(R⁴⁵)-CO-C(R⁴⁶R⁴⁷)-CO-, -O-(CH₂)ₙ-CO-, -N(R⁴⁵)-CO-NH-C(R⁴⁶R⁴⁷)-CO-, -CO-N(R⁴⁵)-C(R⁴⁶R⁴⁷)CO-, -S-(CH₂)ₙ-CO- ou -Z-CO- ;
D représente un radical phénylène éventuellement substitué par un ou plusieurs radicaux identiques ou différents choisis parmi alkyle, alkoxy, OH, nitro, halogène, cyano, ou carboxyl éventuellement estérifié par un radical alkyle ;
Z représente un hétérocycle,
R⁴⁵ représente un atome d'hydrogène ou un radical alkyle,
R⁴⁶ et R⁴⁷ représentent, indépendamment, un atome d'hydrogène, un radical alkyle, aryle ou aralkyle dont les groupements alkyle et aryle sont éventuellement substitués par un ou plusieurs substituants identiques ou différents choisis parmi : le groupe OH, -SH, halogène, nitro, alkyle, alkoxy, alkylthio, aralkoxy, aryl-alkylthio, NR⁴⁸R⁴⁹ et carboxyl éventuellement estérifié par un radical alkyle ;
R⁴⁸ et R⁴⁹ représentent, indépendamment, un atome d'hydrogène, un radical alkyle ou un groupe -COR⁵⁰, ou bien R⁴⁸ et R⁴⁹ forment ensemble avec l'atome d'azote auquel ils sont rattachés un hétérocycle éventuellement substitué,
R⁵⁰ représente un atome d'hydrogène, un radical alkyle, alkoxy ou -NR⁵¹R⁵²,
R⁵¹ et R⁵² représentent, indépendamment, un atome d'hydrogène ou un radical alkyle, ou bien R⁵¹ et R⁵² forment ensemble avec l'atome d'azote auquel ils sont rattachés, un hétérocycle éventuellement substitué ;
n étant un entier compris entre 0 et 6 ;
Yₐ représente -(CH₂)ₚ-, -C(R⁵³R⁵⁴)-(CH₂)ₚ- , -C(R⁵³R⁵⁴)-CO-;
R53 et R⁵⁴ représentent, indépendamment, un atome d'hydrogène, un radical alkyle, un radical aralkyle dont le groupement aryle est éventuellement substitué par un ou plusieurs substituants identiques ou différents choisis parmi : le groupe OH, halogène, nitro, alkyle, alkoxy, -NR⁵⁵R⁵⁶,
R⁵⁵ et R⁵⁶ représentent, indépendamment, un atome d'hydrogène, un radical alkyle ou un groupe -COR⁵⁷, ou bien R⁵⁵ et R⁵⁶ forment ensemble avec l'atome d'azote auquel ils sont rattachés, un hétérocycle éventuellement substitué,
R⁵⁷ représente un atome d'hydrogène, un radical alkyle, alkoxy ou -NR⁵⁸R⁵⁹,
R⁵⁸ et R⁵⁹ représentent, indépendamment, un atome d'hydrogène ou un radical alkyle, ou bien R⁵⁸ et R⁵⁹ forment ensemble avec l'atome d'azote auquel ils sont rattachés un hétérocycle éventuellement substitué ;
p étant un entier compris entre 0 et 6 ;
Hetₐ représente le radical tétrahydrofuran-3-yl,
ainsi que des sels d'addition avec les acides minéraux et organiques ou avec les bases minérales et organiques desdits composés de formule générale (I),
pour la préparation de médicaments pour le traitement de pathologies où les calpaïnes et / ou les formes réactives de l'oxygène sont impliquées.

13. Utilisation de composés de formule (Iₐ) selon la revendication 12, pour la préparation de médicaments pour le traitement de pathologies où les formes réactives de l'oxygène sont impliquées.

14. Utilisation de composés de formule (Iₐ) selon la revendication 12, pour la préparation de médicaments pour le traitement de pathologies où les formes réactives de l'oxygène et les calpaïnes sont impliquées.

15. Utilisation de composés de formule (Iₐ) selon l'une des revendications 12 à 14, pour la préparation de médicaments pour le traitement de pathologies des maladies inflammatoires et immunologiques, des maladies cardio-vasculaires et cérébro-vasculaires, des troubles du système nerveux central ou phériphérique, de l'ostéoporose, des dystrophies musculaires, des maladies prolifératives, de la cataracte, des transplantations d'organes, des maladies auto-immunes et virales, du cancer, et de toutes les pathologies **caractérisées par** une production excessive des ROS et / ou une activation des calpaïnes.

16. Utilisation de composés de formule (Iₐ) selon l'une des revendications 12 à 15, **caractérisés en ce** R¹ représente l'atome d'hydrogène, le radical -OR³ ou oxo.

17. Utilisation de composés de formule (Iₐ) selon l'une des revendications 12 à 16, **caractérisés en ce que** X représente -(CH₂)ₙ-, -(CH₂)ₙ-CO-, -O-(CH₂)ₙ-CO-, -CO-N(R⁴⁵)-D-CO-, -N(R⁴⁵)-CO(CH₂)ₙ-CO-, -N(R⁴⁵)-CO-C(R⁴⁶R⁴⁷)-CO-, -N(R⁴⁵)-CO-NH-C(R⁴⁶R⁴⁷)-CO-, -N(R⁴⁵)-(CH₂)ₙ-CO-, -CO-N(R⁴⁵)-C(R⁴⁶R⁴⁷)-CO ou -Z-CO-.

18. Utilisation de composés de formule (Iₐ) selon la revendication 17, **caractérisés en ce que** R⁴⁵ et R⁴⁷ représentent l'atome d'hydrogène, R⁴⁶ l'atome d'hydrogène, un radical alkyle ou phényle, D le radical phénylène et Z le radical thiazole.

19. Utilisation de composés de formule (Iₐ) selon l'une des revendications 12 à 18, **caractérisés en ce que** R² représente un atome d'hydrogène ou un radical aralkyle, et de préférence benzyle.

20. Utilisation de composés de fonnule (Iₐ) selon l'une des revendications 12 à 19, **caractérisés en ce que** A représente A1 avec W représentant l'atome de soufre,

21. Utilisation de composés de formule (Iₐ) selon l'une des revendications 12 à 20, **caractérisés en ce que** A représente le radical

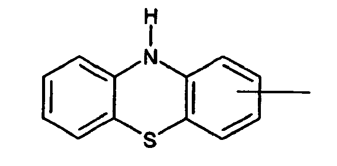

22. Utilisation de composés de formule (Iₐ) selon l'une des revendications 12 à 21, **caractérisés en ce qu'**ils répondent à l'une des formules suivantes :
N-[(1S)-1-({[(3S)-2-hydroxytétrahydro-3-furanyl]amino}carbonyl)-3-méthylbutyl]-10*H-*phénothiazine-2-carboxamide ;
Acétate de (3*S*)-3-({(2*S*)-4-méthyl-2-[(10*H*-phénothiazin-2-ylcarbonyl)-amino]pentanoyl}amino)tétrahydro-2-furanyl ;
N-[(3S)-2-hydroxytétrahydro-3-furanyl]-2-(10*H*-phénothiazin-2-yl)-1,3-thiazole-4-carboxamide ;
N-[4-({[(3S)-2-hydroxytétrahydro-3-furanyl]amino}carbonyl)phényl]-10*H-*phénothiazine-2-carboxamide ;
N-[(1S)-1-({[(3S)-2-hydroxytétrahydro-3-furanyl]amino}carbonyl)-3-méthylbutyl]-10*H*-phénothiazine-1-carboxamide ;
Pivalate de (3S)-3-({(2S)-4-méthyl-2-[(10*H*-phénothiazin-2-ylcarbonyl)amino] pentanoyl}amino)tétrahydro-2-furanyl ;
3,3-diméthylbutanoate de (3S)-3-({(2S)-4-méthyl-2-[(10*H*-phénothiazin-2-ylcarbonyl) amino]pentanoyl}amino)tétrahydro-2-furanyl ;
(3S)-3-({(2S)-4-méthyl-2-[(10*H*-phénothiazin-2-ylcarbonyl)amino]pentanoyl}amino) tétrahydro-2-furanyl benzoate ;
(3S)-3-({(2S)-4-méthyl-2-[(10*H*-phénothiazin-2-ylcarbonyl)amino]pentanoyl}amino) tétrahydro-2-furanyl phénylacetate ;
(3S)-3-({(2S)-4-méthyl-2-[(10*H*-phénothiazin-2-ylcarbonyl)amino]pentanoyl}amino) tétrahydro-2-furanyl (2S)-2-(diméthylamino)-3-phénylpropanoate ;
4-morpholinecarboxylate de (3S)-3-({(2S)-4-méthyl-2-[(10*H*-phénothiazin-2-ylcarbonyl) amino]pentanoyl}amino)tétrahydro-2-furanyl ;

23. Utilisation de composés de formule (Iₐ) selon l'une des revendications 12 à 22, **caractérisés en ce qu'**ils répondent à l'une des formules suivantes :
N-[(1S)-1-({[(3S)-2-hydroxytétrahydro-3-furanyl]amino}carbonyl)-3-méthylbutyl]-10H-phénothiazine-2-carboxamide ;
Acétate de (3*S*)-3-({(2*S*)-4-méthyl-2-[(10*H*-phénothiazin-2-ylcarbonyl)-amino] pentanoyl} amino)tétrahydro-2-furanyl ;
Acétate de (2R,3S)-3-({(2S)-4-méthyl-2-[(10H-phénothiazin-2-ylcarbonyl)amino]-pentanoyl} amino)tétrahydro-2-furanyl ;
Acétate de (2S,3S)-3-({(2S)-4-méthyl-2-[(10H-phénothiazin-2-ylcarbonyl) amino]-pentanoyl} amino)tétrahydro-2-furanyl.

24. A titre de produits industriels, les composés répondant à l'une des formules suivantes :
N-1-(4-anilinophényl)-N-4-[(3S)-2-oxotétrahydro-3-furanyl]succinamide ;
(2S)-2-{[4-(4-anilinoanilino)-4-oxobutanoyl]amino}-4-méthylpentanoate de méthyle ;
N-1-(4-anilinophényl)-N4-[(1S)-1-formyl-3-méthylbutyl]succinamide ;
benzyl 3-(4-anilinoanilino)-3-oxo-2-phénylpropanoate ;
acide 3-(4-anilinoanilino)-3-oxo-2-phénylpropanoïque ;
N-1-(4-anilinophényl)-N-3-[(3S)-2-oxotétrahydro-3-furanyl]-2-phénylmalonamide ;
3-(4-anilinoanilino)dihydro-2(3H)-furanone ;
(2S)-4-méthyl-2-[(10H-phénothiazin-2-ylcarbonyl)amino]pentanoate de méthyle ;
acide (2S)-4-méthyl-2-[(10H-phénothiazin-2-ylcarbonyl)amino]pentanoïque ;
N-[(1S)-3-méthyl-1-({[(3S)-2-oxotétrahydro-3-furanyl]amino}carbonyl)butyl]-10H-phénothiazine-2-carboxamide ;
2-(10H-phénothiazin-2-yl)-1,3-thiazole-4-carboxylate d'éthyle;
acide 2-(10H-phénothiazin-2-yl)-1,3-thiazole-4-carboxylique ;
N-[(3S)-2-oxotétrahydro-3-furanyl]-2-(10*H*-phénothiazin-2-yl)-1,3-thiazole-4-carboxamide ;
4-[(10H-phénothiazin-2-ylcarbonyl)amino]benzoate de méthyle ;
acide 4-[(10H-phénothiazin-2-ylcarbonyl)amino]benzoïque ;
N-[4-({[(3S)-2-oxotétrahydro-3-furanyl]amino}carbonyl)phényl]-10H-phénothiazine-2-carboxamide ;
(2S)-4-méthyl-2-[(10H-phénothiazin-1-ylcarbonyl)amino]pentanoate de méthyle ;
acide (2S)-4-méthyl-2-[(10H-phénothiazin-1-ylcarbonyl)amino]pentanoïque ;
N-[(1S)-1-({[(3S)-2-oxotétrahydro-3-furanyl]amino}carbonyl)-3-méthylbutyl]-10H-phénothiazine-1-carboxamide ;
*N*-[(1*S*)-1-(1,4-dioxa-7-azaspiro[4.4]non-7-ylcarbonyl)-3-méthylbutyl]-10*H* phénothiazine-2-carboxamide ;
2-(3,5-di-*tert*-butyl-4-hydroxyphénoxy)-*N*-[(3S)-2-oxotétrahydro-3-furanyl] acétamide
5-nitro-1-propylindoline ;
1-propyl-2,3-dihydro-1*H*-indol-5-ylamine;
3-oxo-2-phényl-N-(1-propyl-2,3-dihydro-1H-indol-5-yl)-béta-alanine ;
N¹-[(3S)-2-oxotétrahydro-3-furanyl]-2-phényl-N3-(1-propyl-2,3-dihydro-1H-indol-5-yl)malonamide;
*N*-(2-anilinophényl)-*N*'-[(3*S*)-2-oxotétrahydro-3-furanyl]urée ;
oxo[(1-propyl-2,3-dihydro-1H-indol-5-yl)amino]acétate d'éthyle ;
acide oxo[(1-propyl-2,3-dihydro-1H-indol-5-yl)amino]acétique ;
(2S)-2-({[(2R)-6-hydroxy-2,5,7,8-tétramethyl-3,4-dihydro-2H-chromen-2-yl]carbonyl}amino)-3-phenylpropanoate de méthyle ;
(2R)-N-[(1S)-1-benzyl-2-oxoéthyl]-6-hydroxy-2,5,7,8-tétramethyl-3,4-dihydro-2H-chromène-2-carboxamide ;
5-methyl 1,5-indolinedicarboxylate de *tert*-butyle ;
acide 1-(*tert*-butoxycarbonyl)-5-indolinecarboxylique ;
5-({[(3S)-2-oxotétrahydro-3-furanyl]amino}carbonyl)-1-indolinecarboxylate de *tert*-butyle ;
5-({[(3S)-2-hydroxytétrahydro-3-furanyl]amino}carbonyl)-1-indolinecarboxylate de *tert*-butyle.

## Claims

1. Compounds of general formula (I) 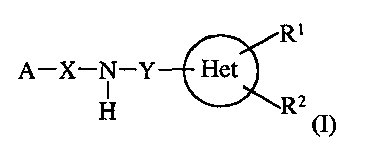
in racemic, enantiomeric, diastereoisomeric form or all combinations of these forms, in which
R¹ represents a hydrogen atom, an -OR³, -SR³, oxo or cyclic acetal radical,
in which R³ represents a hydrogen atom, an alkyl, arylalkyl, heterocycloalkylcarbonyl, alkylcarbonyl, arylcarbonyl or aralkylcarbonyl radical,
in which the alkyl, aryl or heterocycloalkyl radicals are optionally substituted by one or more identical or different substituents chosen from: alkyl, OH, alkoxy, nitro, cyano, halogen or -NR⁴R⁵;
R⁴ and R⁵ represent, independently, a hydrogen atom or an alkyl radical, or R⁴ and R⁵ together with the nitrogen atom to which they are attached form an optionally substituted heterocycle,
R² represents a hydrogen atom, an alkyl, aryl or aralkyl radical, the aryl group being optionally substituted by one or more identical or different radicals chosen from: -OR⁶, -NR⁷R⁸, halogen, cyano, nitro or alkyl,
in which R⁶, R⁷ and R⁸ represent, independently, a hydrogen atom, an alkyl, aryl, aralkyl, alkylcarbonyl, arylcarbonyl or aralkylcarbonyl radical;
A represents an A1 radical 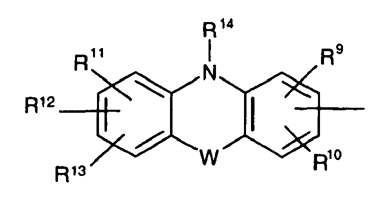
in which R⁹, R¹⁰, R¹¹, R¹², R¹³ represent, independently, a hydrogen atom, a halogen, the OH group, an alkyl, alkoxy, cyano, nitro or -NR¹⁵R¹⁶ radical,
R¹⁵ and R¹⁶ represent, independently, a hydrogen atom, an alkyl radical or a -COR¹⁷ group, or R¹⁵ and R¹⁶ together with the nitrogen atom to which they are attached form an optionally substituted heterocycle,
R¹⁷ represents a hydrogen atom, an alkyl, alkoxy or -NR¹⁸R¹⁹ radical,
R¹⁸ and R¹⁹ represent, independently, a hydrogen atom or an alkyl radical, or R¹⁸ and R¹⁹ together with the nitrogen atom to which they are attached form an optionally substituted heterocycle,
R¹⁴ represents a hydrogen atom, an alkyl radical or a -COR²⁰ group,
R²⁰ represents a hydrogen atom, an alkyl, alkoxy, aryl, aralkyl, heterocycloalkyl or -NR²¹R²² radical,
in which the alkyl, aryl or heterocycloalkyl radicals are optionally substituted by one or more identical or different substituents chosen from: alkyl, OH, alkoxy, nitro, cyano, halogen or -NR⁴R⁵;
R²¹ and R²² represent, independently, a hydrogen atom or an alkyl radical, or R²¹ and R²² together with the nitrogen atom to which they are attached form an optionally substituted heterocycle,
W represents a bond, O or S or also an -NR²³ radical, in which R²³ represents a hydrogen atom or an alkyl radical;
X represents -(CH₂)ₙ-, -(CH₂)ₙ-CO-, -N(R⁴⁵)-CO-(CH₂)ₙ-CO-, -N(R⁴⁵)-CO-D-CO-, -CO-N(R⁴⁵)-D-CO-, -CO-D-CO-, -CH=CH-(CH₂)ₙ-CO-, -N(R⁴⁵)-(CH₂)ₙ-CO-, -N(R⁴⁵)-CO-C(R⁴⁶R⁴⁷)-CO-, -O-(CH₂)ₙ-CO-, -N(R⁴⁵)-CO-NH-C(R⁴⁶R⁴⁷)-CO-, -CO-N(R⁴⁵)-C(R⁴⁶R⁴⁷)-CO-, -S-(CH₂)ₙ-CO- or -Z-CO-;
D represents a phenylene radical optionally substituted by one or more identical or different radicals chosen from alkyl, alkoxy, OH, nitro, halogen, cyano, or carboxyl optionally esterified by an alkyl radical;
Z represents a heterocycle,
R⁴⁵ represents a hydrogen atom or an alkyl radical,
R⁴⁶ and R⁴⁷ represent, independently, a hydrogen atom, an alkyl, aryl or aralkyl radical the alkyl and aryl groups of which are optionally substituted by one or more identical or different substituents chosen from: the OH, -SH, halogen, nitro, alkyl, alkoxy, alkylthio, aralkoxy, aryl-alkylthio, -NR⁴⁸R⁴⁹ and carboxyl group optionally esterified by an alkyl radical;
R⁴⁸ and R⁴⁹ represent, independently, a hydrogen atom, an alkyl radical or a -COR⁵⁰ group, or R⁴⁸ and R⁴⁹ together with the nitrogen atom to which they are attached form an optionally substituted heterocycle,
R⁵⁰ represents a hydrogen atom, an alkyl, alkoxy or -NR⁵¹R⁵² radical,
R⁵¹ and R⁵² represent, independently, a hydrogen atom or an alkyl radical, or R⁵¹ and R⁵² together with the nitrogen atom to which they are attached, form an optionally substituted heterocycle;
n being an integer comprised between 0 and 6;
Y represents -(CH₂)ₚ-, -C(R⁵³R⁵⁴)-(CH₂)ₚ- , -C(R⁵³R⁵⁴)-CO-;
R⁵³ and R⁵⁴ represent, independently, a hydrogen atom, an alkyl radical, an aralkyl radical the aryl group of which is optionally substituted by one or more identical or different substituents chosen from: the OH, halogen, nitro, alkyl, alkoxy, -NR⁵⁵R⁵⁶ group,
R⁵⁵ and R⁵⁶ represent, independently, a hydrogen atom, an alkyl radical or a -COR⁵⁷ group, or R⁵⁵ and R⁵⁶ together with the nitrogen atom to which they are attached, form an optionally substituted heterocycle,
R⁵⁷ represents a hydrogen atom, an alkyl, alkoxy or -NR⁵⁸R⁵⁹ radical,
R⁵⁸ and R⁵⁹ represent, independently, a hydrogen atom or an alkyl radical, or R⁵⁸ and R⁵⁹ together with the nitrogen atom to which they are attached form an optionally substituted heterocycle;
p being an integer comprised between 0 and 6;
Het represents the tetrahydrofuran-3-yl radical,
as well as the addition salts with mineral and organic acids or with mineral and organic bases of said compounds of general formula (I),
with the exception of the compounds of formula (I) in which when Het represents tetrahydrofuran or tetrahydropyran, R¹ represents the OR³ radical with R³ representing a hydrogen atom, an alkyl, arylalkyl, heterocycloalkylcarbonyl radical the heterocycloalkyl radical of which is connected by a carbon atom, alkylcarbonyl, arylcarbonyl or aralkylcarbonyl radical, R² represents a hydrogen and Y represents the -(CH₂)ₚ- radical with p = 0, then X represents -CO-N(R⁴⁵)-C(R⁴⁶R⁴⁷)-CO- with R⁴⁵ = R⁴⁶ = H.

2. Compounds according to claim 1, **characterized in that** R¹ represents the hydrogen atom, the -OR³ or oxo radical.

3. Compounds according to one of the previous claims, **characterized in that** X represents -(CH₂)ₙ-, -(CH₂)ₙ-CO-, -O-(CH₂)ₙ-CO-, -CO-N(R⁴⁵)-D-CO-, -N(R⁴⁵)-CO-(CH₂)ₙ-CO-, -N(R⁴⁵)-CO-C(R⁴⁶R⁴⁷)-CO-, -N(R⁴⁵)-CO-NH-C(R⁴⁶R⁴⁷)-CO-, -N(R⁴⁵)-(CH₂)n-CO-, -CO-N(R⁴⁵)-C(R⁴⁶R⁴⁷)-CO or -Z-CO-.

4. Compounds according to claim 3, **characterized in that** R⁴⁵ and R⁴⁷ represent the hydrogen atom, R⁴⁶ represents the hydrogen atom, an alkyl or phenyl radical, D represents the phenylene radical and Z represents the thiazole radical.

5. Compounds according to one of the previous claims, **characterized in that** R² represents a hydrogen atom or an aralkyl radical, and preferably benzyl.

6. Compounds according to one of the previous claims, **characterized in that** A represents A1 with W representing the sulphur atom.

7. Compounds according to one of the previous claims, **characterized in that** A represents the radical

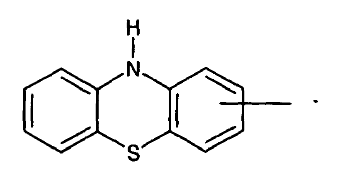

8. Compounds corresponding to one of the following formulae :
N-[(1S)-1-({[(3S)-2-hydroxytetrahydro-3-furanyl]amino}carbonyl)-3-methylbutyl)-10*H-*phenothiazine-2-carboxamide;
(3*S*)-3-({(2*S*)-4-methyl-2-[(10*H*-phenothiazin-2-ylcarbonyl)-amino]pentanoyl} amino)tetrahydro-2-furanyl acetate;
N-[(3S)-2-hydroxytetrahydro-3-furanyl]-2-(10*H*-phenothiazin-2-yl)-1,3-thiazol-4-carboxamide;
N-[4-({[(3S)-2-hydroxytetrahydro-3-furanyl]amino}carbonyl)phenyl]-10*H-*phenothiazine-2-carboxamide;
N-[(1S)-1-({{(3S)-2-hydroxytetrahydro-3-furanyl]amino}carbonyl)-3-methylbutyl]-10*H*-phenothiazine-1-carboxamide;
(3S)-3-({(2S)-4-methyl-2-[(10*H*-phenothiazin-2-ylcarbonyl)amino] pentanoyl}amino)tetrahydro-2-furanyl pivalate;
(3S)-3-({(2S)-4-methyl-2-[(10*H*-phenothiazin-2-ylcarbonyl)amino]pentanoyl}amino)-tetrahydro-2-furanyl 3,3-dimethylbutanoate;
(3S)-3-({(2S)-4-methyl-2-[(10*H*-phenothiazin-2-ylcarbonyl)amino]pentanoyl}amino) tetrahydro-2-furanyl benzoate;
(3S)-3-({(2S)-4-methyl-2-[(10*H*-phenothiazin-2-ylcarbonyl)amino]pentanoyl}amino) tetrahydro-2-furanyl phenylacetate;
(3*S*)-3-({(2*S*)-4-methyl-2-[(10*H*-phenothiazin-2-ylcarbonyl)amino]pentanoyl}amino) tetrahydro-2-furanyl (2*S*)-2-(dimethylamino)-3-phenylpropanoate;
(3*S*)-3-({(25)-4-methyl-2-[(10*H*-phenothiazin-2-ylcarbonyl)amino]pentanoyl}amino) tetrahydro-2-furanyl 4-morpholinecarboxylate;

9. Compounds corresponding to one of the following formulae :
N-[(1S)-1-({[(3S)-2-hydroxytetrahydro-3-furanyl]amino}carbonyl)-3-methylbutyl]-10*H-*phenothiazine-2-carboxamide;
(3S)-3-({(2S)-4-methyl-2-[(10*H*-phenothiazin-2-ylcarbonyl)-amino]pentanoyl}amino) tetrahydro-2-furanyl acetate ;
(2R,3S)-3-({(2S)-4-methyl-2-[(10*H*-phenothiazin-2-ylcarbonyl)-amino]pentanoyl} amino) tetrahydro-2-furanyl acetate;
(2S,3S)-3-({(2S)-4-methyl-2-[{10*H*-phenothiazin-2-ylcarbonyl)-amino]pentanoyla}mino) tetrahydro-2-furanyl acetate ; as well as the addition salts with mineral and organic acids or with mineral and organic bases of said compounds.

10. As medicaments, compounds according to one of claims 1 to 9.

11. Pharmaceutical compositions comprising, as active ingredient, at least one medicament as defined in claim 10.

12. Use of compounds of formula (Iₐ) as defined above, 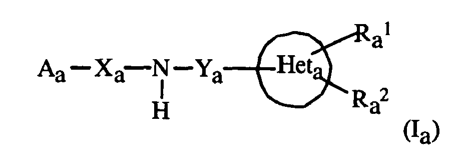
in racemic, enantiomeric, diastereoisomeric form or all combinations of these forms, in which
Rₐ¹ represents a hydrogen atom, an -OR³, -SR³, oxo or cyclic acetal radical,
in which R³ represents a hydrogen atom, an alkyl, arylalkyl, heterocycloalkylcarbonyl, alkylcarbonyl, arylcarbonyl or aralkylcarbonyl radical,
in which the alkyl, aryl or heterocycloalkyl radicals are optionally substituted by one or more identical or different substituents chosen from: alkyl, OH, alkoxy, nitro, cyano, halogen or -NR⁴R⁵;
R⁴ and R⁵ represent, independently, a hydrogen atom or an alkyl radical, or R⁴ and R⁵ together with the nitrogen atom to which they are attached form an optionally substituted heterocycle,
Rₐ² represents a hydrogen atom, an alkyl, aryl or aralkyl radical, the aryl group being optionally substituted by one or more identical or different radicals chosen from: -OR⁶, -NR⁷R⁸, halogen, cyano, nitro or alkyl,
in which R⁶, R⁷ and R⁸ represent, independently, a hydrogen atom, an alkyl, aryl, aralkyl, alkylcarbonyl, arylcarbonyl or aralkylcarbonyl radical;
Aₐ represents an A1 radical 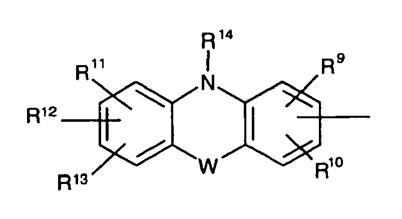
in which R⁹, R¹⁰, R¹¹, R¹², R¹³ represent, independently, a hydrogen atom, a halogen, the OH group, an alkyl, alkoxy, cyano, nitro or -NR¹⁵R¹⁶ radical,
R¹⁵ and R¹⁶ represent, independently, a hydrogen atom, an alkyl radical or a -COR¹⁷ group, or R¹⁵ and R¹⁶ together with the nitrogen atom to which they are attached form an optionally substituted heterocycle,
R¹⁷ represents a hydrogen atom, an alkyl, alkoxy or -NR¹⁸R¹⁹ radical,
R¹⁸ and R¹⁹ represent, independently, a hydrogen atom or an alkyl radical, or R¹⁸ and R¹⁹ together with the nitrogen atom to which they are attached form an optionally substituted heterocycle,
R¹⁴ represents a hydrogen atom, an alkyl radical or a -COR²⁰ group,
R²⁰ represents a hydrogen atom, an alkyl, alkoxy, aryl, aralkyl, heterocycloalkyl or -N-R²¹R²² radical,
in which the alkyl, aryl or heterocycloalkyl radicals are optionally substituted by one or more identical or different substituents chosen from: alkyl, OH, alkoxy, nitro, cyano, halogen or -NR⁴R⁵ ;
R²¹ and R²² represent, independently, a hydrogen atom or an alkyl radical, or R²¹ and R²² together with the nitrogen atom to which they are attached form an optionally substituted heterocycle,
W represents a bond, O or S or also an -NR²³ radical, in which R²³ represents a hydrogen atom or an alkyl radical;
Xₐ represents -(CH₂)ₙ-, -(CH₂)ₙ CO-, -N(R⁴⁵)-CO-(CH₂)ₙ-CO-, -N(R⁴⁵)-CO-D-CO-, -CO-N(R⁴⁵)-D-CO-, -CO-D-CO-, -CH=CH-(CH₂)ₙ-CO-, -N(R⁴⁵)-(CH₂)ₙ-CO-, -N(R⁴⁵)-CO-C(R⁴⁶R⁴⁷)-CO-, -O-(CH₂)ₙ-CO-, -N(R⁴⁵)-CO-NH-C(R⁴⁶R⁴⁷)-CO-, -CO-N(R⁴⁵)-C(R⁴⁶R⁴⁷)-CO-, -S-(CH₂)ₙ-CO- or -Z-CO-;
D represents a phenylene radical optionally substituted by one or more identical or different radicals chosen from alkyl, alkoxy, OH, nitro, halogen, cyano, or carboxyl optionally esterified by an alkyl radical;
Z represents a heterocycle,
R⁴⁵ represents a hydrogen atom or an alkyl radical,
R⁴⁶ and R⁴⁷ represent, independently, a hydrogen atom, an alkyl, aryl or aralkyl radical the alkyl and aryl groups of which are optionally substituted by one or more identical or different substituents chosen from: the OH, -SH, halogen, nitro, alkyl, alkoxy, alkylthio, aralkoxy, aryl-alkylthio, NR⁴⁸R⁴⁹ and carboxyl group optionally esterified by an alkyl radical;
R⁴⁸ and R⁴⁹ represent, independently, a hydrogen atom, an alkyl radical or a -COR⁵⁰ group, or R⁴⁸ and R⁴⁹ together with the nitrogen atom to which they are attached form an optionally substituted heterocycle,
R⁵⁰ represents a hydrogen atom, an alkyl, alkoxy or -NR⁵¹R⁵² radical,
R⁵¹ and R⁵² represent, independently, a hydrogen atom or an alkyl radical, or R⁵¹ and R⁵² together with the nitrogen atom to which they are attached form an optionally substituted heterocycle;
n being an integer comprised between 0 and 6;
Yₐ represents -(CH₂)ₚ- , -C(R⁵³R⁵⁴)-(CH₂)ₚ- , -C(R⁵³R⁵⁴)-CO- ;
R⁵³ and R⁵⁴ represent, independently, a hydrogen atom, an alkyl radical, an aralkyl radical the aryl group of which is optionally substituted by one or more identical or different substituents chosen from: the OH group, halogen, nitro, alkyl, alkoxy, -NR⁵⁵R⁵⁶,
R⁵⁵ and R⁵⁶ represent, independently, a hydrogen atom, an alkyl radical or a -COR⁵⁷ group, or R⁵⁵ and R⁵⁶ together with the nitrogen atom to which they are attached form an optionally substituted heterocycle,
R⁵⁷ represents a hydrogen atom, an alkyl, alkoxy or -NR⁵⁸R⁵⁹ radical,
R⁵⁸ and R⁵⁹ represent, independently, a hydrogen atom or an alkyl radical, or R⁵⁸ and R⁵⁹ together with the nitrogen atom to which they are attached form an optionally substituted heterocycle;
p being an integer comprised between 0 and 6 ;
Hetₐ represents tetrahydrofuran-3-yl radical,
as well as addition salts with mineral and organic acids or with mineral and organic bases of said compounds of general formula (I),
for the preparation of medicaments for the treatment of pathologies where calpains and / or reactive oxygen species are involved.

13. Use of compounds of formula (Iₐ) according to claim 12, for the preparation of medicaments for the treatment of pathologies involving reactive oxygen species.

14. Use of compounds of formula (Iₐ) according to claim 12, for the preparation of medicaments for the treatment of pathologies involving reactive oxygen species and calpains.

15. Use of compounds of formula (Iₐ) according to one of claims 12 to 14, for the preparation of medicaments for the treatment of pathologies of inflammatory and immunological diseases, cardiovascular and cerebrovascular diseases, disorders of the central or peripheral nervous system, osteoporosis, muscular dystrophies, proliferative diseases, cataracts, organ transplants, auto-immune and viral diseases, cancer, and all pathologies **characterized by** the excessive production of ROS and / or activation of calpains.

16. Use of compounds of formula (Iₐ) according to one of claims 12 to 15, **characterized in that** R¹ represents the hydrogen atom, the -OR³ or oxo radical.

17. Use of compounds of formula (Iₐ) according to one of claims 12 to 16, **characterized in that** X represents -(CH₂)ₙ- , -(CH₂)ₙ-CO-, -O-(CH₂)ₙ-CO-, -CO-N(R⁴⁵)-D-CO-, -N(R⁴⁵)-CO-(CH₂)ₙ-CO-, -N(R⁴⁵)-CO-C(R⁴⁶R⁴⁷)-CO-, -N(R⁴⁵)-CO-NH-C(R⁴⁶R⁴⁷)-CO-, -N(R⁴⁵)-(CH₂)ₙ-CO-, -CO-N(R⁴⁵)-C(R⁴⁶R⁴⁷)-CO or -Z-CO-.

18. Use of compounds of formula (Iₐ) according to claim 17, **characterized in that** R⁴⁵ and R⁴⁷ represent the hydrogen atom, R⁴⁶ represents the hydrogen atom, an alkyl or phenyl radical, D represents the phenylene radical and Z represents the thiazole radical.

19. Use of compounds of formula (Iₐ) according to one of claims 12 to 18, **characterized in that** R² represents a hydrogen atom or an aralkyl radical, and preferably benzyl.

20. Use of compounds of formula (Iₐ) according to one of claims 12 to 19, **characterized in that** A represents A1 with W representing the sulphur atom.

21. Use of compounds of formula (Iₐ) according to one of claims 12 to 20, **characterized in that** A represents the radical

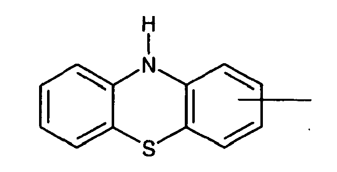

22. Use of compounds of formula (Iₐ) according to one of claims 12 to 21, **characterized in that** they correspond to one of the following formulae :
N-[(1S)-1-({[(3S)-2-hydroxytetrahydro-3-furanyl]amino}carbonyl)-3-methylbutyl]-10*H-*phenothiazine-2-carboxamide;
(3*S*)-3-({(2*S*)-4-methyl-2-[(10*H*-phenothiazin-2-ylcarbonyl)-amino]pentanoyl} amino)tetrahydro-2-furanyl acetate;
N-[(3S)-2-hydroxytetrahydro-3-furanyl]-2-(10*H*-phenothiazin-2-yl)-1,3-thiazol-4-carboxamide;
N-[4-({[(3S)-2-hydroxytetrahydro-3-furanyl]amino}carbonyl)phenyl]-10*H-*phenothiazine-2-carboxamide;
N-[(1S)-1-([(3S)-2-hydroxytetrahydro-3-furanyl]amino}carbonyl)-3-methylbutyl]-10*H*-phenothiazine-1-carboxamide;
(3S)-3-({(2S)-4-methyl-2-[(10*H*-phenothiazin-2-ylcarbonyl)amino] pentanoyl}amino)tetrahydro-2-furanyl pivalate;
(3S)-3-({(2S)-4-methyl-2-[(10*H*-phenothiazin-2-ylcarbonyl)amino]pentanoyl}amino) tetrahydro-2-furanyl 3,3-dimethylbutanoate;
(3S)-3-({(2S)-4-methyl-2-[(10*H*-phenothiazin-2-ylcarbonyl)amino]pentanoyl}amino) tetrahydro-2-furanyl benzoate;
(3S)-3-({(2S)-4-methyl-2-[(10*H*-phenothiazin-2-ylcarbonyl)amino]pentanoyl}amino) tetrahydro-2-furanyl phenylacetate;
(3*S*)-3-({(2*S*)-4-methyl-2-[(10*H*-phenothiazin-2-ylcarbonyl)amino]pentanoyl} amino) tetrahydro-2-furanyl (2*S*)-2-(dimethylamino)-3-phenylpropanoate;
(3S)-3-({(2S)-4-methyl-2-[(10*H*-phenothiazin-2-ylcarbonyl) amino]pentanoyl}amino) tetrahydro-2-furanyl 4-morpholinecarboxylate ;

23. Use of compounds of formula (Iₐ) according to one of claims 12 to 22, **characterized in that** they correspond to one of the following formulae
N-[(1S)-1-({[(3S)-2-hydroxytetrahydro-3-furanyl]amino}carbonyl)-3-methylbutyl]-10*H-*phenothiazine-2-carboxamide;
(3S)-3-({(2S)-4-methyl-2-[(10*H*-phenothiazin-2-ylcarbonyl)-amino]pentanoyl}amino) tetrahydro-2-furanyl acetate;
(2R,3S)-3-({(2S)-4-methyl-2-[(10*H*-phenothiazin-2-ylcarbonyl)-amino]pentanoyl} amino) tetrahydro-2-furanyl acetate;
(2S,3S)-3-({(2S)-4-methyl-2-[(10*H*-phenothiazin-2-ylcarbonyl)-amino]pentanoyl}amino) tetrahydro-2-furanyl acetate.

24. As industrial products, the compounds corresponding to one of the following formulae:
N-1-(4-anilinophenyl)-N-4-[(3S)-2-oxotetrahydro-3-furanyl]succinamide;
methyl (2S)-2-{[4-(4-anilinoanilino)-4-oxobutanoyl]amino}-4-methylpentanoate;
N-1-(4-anilinophenyl)-N4-[(1S)-1-formyl-3-methylbutyl]succinamide;
benzyl 3-(4-anilinoanilino)-3-oxo-2-phenylpropanoate;
3-(4-anilinoanilino)-3-oxo-2-phenylpropanoic acid;
N-1-(4-anilinophenyl)-N-3-[(3S)-2-oxotetrahydro-3-furanyl]-2-phenylmalonamide;
3-(4-anilinoanilino)dihydro-2(3H)-furanone;
methyl (2S)-4-methyl-2-[(10H-phenothiazin-2-ylcarbonyl)amino]pentanoate;
(2S)-4-methyl-2-[(10H-phenothiazin-2-ylcarbonyl)amino]pentanoic acid;
N-[(1S)-3-methyl-1-({[(3S)-2-oxotetrahydro-3-furanyl]amino}carbonyl)butyl]-10H-phenothiazine-2-carboxamide;
ethyl 2-(10H-phenothiazin-2-yl)-1,3-thiazol-4-carboxylate;
2-(10H-phenothiazin-2-yl)-1,3-thiazol-4-carboxylic acid;
N-[(3S)-2-oxotetrahydro-3-furanyl]-2-(10H-phenothiazin-2-yl)-1,3-thiazol-4-carboxamide;
methyl 4-[(10H-phenothiazin-2-ylcarbonyl)amino]benzoate;
4-[(10H-phenothiazin-2-ylcarbonyl)amino]benzoic acid;
N-[4-({[(3S)-2-oxotetrahydro-3-furanyl]amino}carbonyl)phenyl]-10H-phenothiazine-2-carboxamide;
methyl (2S)-4-methyl-2-[(10H-phenothiazin-1-ylcarbonyl)amino]pentanoate;
(2S)-4-methyl-2-[(10H-phenothiazin-1-ylcarbonyl)amino]pentanoic acid;
N-[(1S)-1-({[(3S)-2-oxotetrahydro-3-furanyl]amino}carbonyl)-3-methylbutyl]-10H-phenothiazine-1-carboxamide;
*N*[(1*S*)-1-(1,4-dioxa-7-azaspiro[4.4]non-7-ylcarbonyl)-3-methylbutyl]-10*H-*phenothiazine-2-carboxamide;
2-(3,5-di-*tert*-butyl-4-hydroxyphenoxy)-*N*-[(3*S*)-2-oxotetrahydro-3-furanyl] acetamide;
5-nitro-1-propylindoline;
1-propyl-2,3-dihydro-1*H*-indol-5-ylamine;
3-oxo-2-phenyl-N-(1-propyl-2,3-dihydro-1H-indol-5-yl)-beta-alanine;
N¹-[(3S)-2-oxotetrahydro-3-furanyl]-2-phenyl-N³-(1-propyl-2,3-dihydro-1H-indol-5-yl)malonamide;
*N*-(2-anilinophenyl)-*N*'-[(3*S*)-2-oxotetrahydro-3-furanyl]urea;
ethyl oxo[(1-propyl-2,3-dihydro-1H-indol-5-yl)amino] acetate;
oxo[(1-propyl-2,3-dihydro-1H-indol-5-yl)amino]acetic acid;
methyl (2S)-2-({[(2R)-6-hydroxy-2,5,7,8-tetramethyl-3,4-dihydro-2H-chromen-2-yl]carbonyl }amino)-3-phenylpropanoate;
(2R)-N-[(1S)-1-benzyl-2-oxoethyl]-6-hydroxy-2,5,7,8-tetramethyl-3,4-dihydro-2H-chromene-2-carboxamide;
*tert*-butyl 5-methyl 1,5-indolinedicarboxylate;
1-(*tert*-butoxycarbonyl)-5-indolinecarboxylic acid;
*tert*-butyl 5-({[(3S)-2-oxotetrahydro-3-furanyl]amino}carbonyl)-1-indoline carboxylate;
*tert*-butyl 5-({[(3S)-2-hydroxytetrahydro-3-furanyl]amino}carbonyl)-1-indoline carboxylate.

## Patentansprüche

1. Verbindungen der allgemeinen Formel (I) 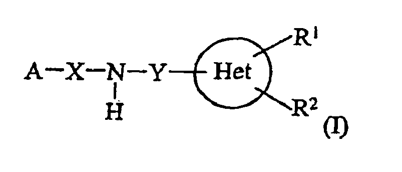
in Form von Racemat, Enantiomeren, Diastereoisomeren und allen Kombinationen dieser Formen, in der
R¹ ein Wasserstoffatom, einen Rest -OR³, -SR³, Oxo oder ein cyclisches Acetal darstellt,
wobei R³ ein Wasserstoffatom, einen der Reste Alkyl, Arylalkyl, Heterocycloalkylcarbonyl, Alkylcarbonyl, Arylcarbonyl oder Aralkylcarbonyl darstellt,
wobei die Reste Alkyl, Aryl oder Heterocycloalkyl ggf. durch einen oder mehrere gleiche oder verschiedene Substituenten substituiert sind, die ausgewählt sind aus: Alkyl, OH, Alkoxy, Nitro, Cyano, Halogen oder -NR⁴R⁵;
R⁴ und R⁵ unabhängig voneinander ein Wasserstoffatom oder einen Alkylrest darstellen oder R⁴ und R⁵ zusammen mit dem Stickstoffatom, an das sie gebunden sind, einen ggf. substituierten Heterocyclus bilden,
R² ein Wasserstoffatom, einen Alkyl-, Aryl- oder Aralkylrest darstellt, wobei die Arylgruppe ggf. durch einen oder mehrere gleiche oder verschiedene Reste substituiert ist, die ausgewählt sind aus: -OR⁶, -NR⁷R⁸, Halogen, Cyano, Nitro oder Alkyl,
wobei R⁶, R⁷ und R⁸ unabhängig voneinander ein Wasserstoffatom, einen der Reste Alkyl, Aryl, Aralkyl, Alkylcarbonyl, Arylcarbonyl oder Aralkylcarbonyl darstellen;
A einen Rest A1 darstellt 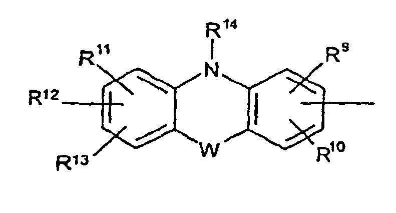
bei dem R⁹, R¹⁰, R¹¹, R¹², R¹³ unabhängig voneinander ein Wasserstoffatom, ein Halogen, eine OH-Gruppe, einen der Reste Alkyl, Alkoxy, Cyano, Nitro oder -NR¹⁵R¹⁶ darstellen,
R¹⁵ und R¹⁶ unabhängig voneinander ein Wasserstoffatom, einen Alkylrest oder eine -COR¹⁷-Gruppe darstellen oder R¹⁵ und R¹⁶ zusammen mit dem Stickstoffatom, an das sie gebunden sind, einen ggf. substituierten Heterocyclus bilden,
R¹⁷ ein Wasserstoffatom, einen der Reste Alkyl, Alkoxy oder -NR¹⁸R¹⁹ darstellt,
R¹⁸ und R¹⁹ unabhängig voneinander ein Wasserstoffatom oder einen Alkylrest darstellen oder R¹⁸ und R¹⁹ zusammen mit dem Stickstoffatom, an das sie gebunden sind, einen ggf. substituierten Heterocyclus bilden,
R¹⁴ ein Wasserstoffatom, einen Alkylrest oder eine -COR²⁰-Gruppe darstellt,
R²⁰ ein Wasserstoffatom, einen der Reste Alkyl, Alkoxy, Aryl, Aralkyl, Heterocycloalkyl oder -NR²¹R²² darstellt,
wobei die Reste Alkyl, Aryl oder Heterocycloalkyl ggf. durch einen oder mehrere gleiche oder verschiedene Substituenten substituiert sind, die ausgewählt sind aus: Alkyl, OH, Alkoxy, Nitro, Cyano, Halogen oder -NR⁴R⁵;
R²¹ und R²² unabhängig voneinander ein Wasserstoffatom oder einen Alkylrest darstellen oder R²¹ und R²² zusammen mit dem Stickstoffatom, an das sie gebunden sind, einen ggf. substituierten Heterocyclus bilden,
W eine Bindung, O oder S oder auch einen -NR²³-Rest darstellt, wobei R²³ ein Wasserstoffatom oder einen Alkylrest darstellt;
X -(CH₂)ₙ-, -(CH₂)ₙ-CO-, -N(R⁴⁵)-CO-(CH₂)ₙ-CO-, -N (R⁴⁵) -CO-D-CO-, -CO-N (R⁴⁵) -D-CO-, -CO-D-CO-, -CH=CH- (CH₂)ₙ-CO-, -N (R⁴⁵) - (CH₂)ₙ-CO-, -N (R⁴⁵) -CO-C (R⁴⁶R⁴⁷) -CO-, -O- (CH₂)ₙ-CO-, -N (R⁴⁵) -CO-NH-C (R⁴⁶R⁴⁷) -CO-, -CO-N (R⁴⁵) -C(R⁴⁶R⁴⁷) -CO-, -S-(CH₂)ₙ-CO- oder -Z-CO- darstellt;
D einen Phenylenrest darstellt, der ggf. mit ein oder mehreren gleichen oder verschiedenen Resten substituiert ist, die aus Alkyl, Alkoxy, OH, Nitro, Halogen, Cyano oder einem ggf. durch einen Alkylrest veresterten Carboxyl ausgewählt sind;
Z einen Heterocyclus darstellt,
R⁴⁵ ein Wasserstoffatom oder einen Alkylrest darstellt,
R⁴⁶ und R⁴⁷ unabhängig voneinander ein Wasserstoffatom, einen der Reste Alkyl, Aryl oder Aralkyl darstellen, wobei die Gruppen Alkyl und Aryl ggf. durch einen oder mehrere gleiche oder verschiedene Substituenten substituiert sind, die ausgewählt sind aus: eine OH-Gruppe, -SH, Halogen, Nitro, Alkyl, Alkoxy, Alkylthio, Aralkoxy, Arylalkylthio, -NR⁴⁸R⁴⁹ und ggf. durch einen Alkylrest verestertes Carboxyl;
R⁴⁸ und R⁴⁹ unabhängig voneinander ein Wasserstoffatom, einen Alkylrest oder eine -COR⁵⁰-Gruppe darstellen oder R⁴⁸ und R⁴⁹ zusammen mit dem Stickstoffatom, an das sie gebunden sind, einen ggf. substituierten Heterocyclus bilden,
R⁵⁰ ein Wasserstoffatom, einen der Reste Alkyl, Alkoxy oder -NR⁵¹R⁵² darstellt,
R⁵¹ und R⁵² unabhängig voneinander ein Wasserstoffatom oder einen Alkylrest darstellen oder R⁵¹ und R⁵² zusammen mit dem Stickstoffatom, an das sie gebunden sind, einen ggf. substituierten Heterocyclus bilden;
wobei n eine ganze Zahl zwischen 0 und 6 ist;
Y-(CH₂)ₚ-, -C(R⁵³R⁵⁴)-(CH₂)ₚ-, -C(R⁵³R⁵⁴)-CO- darstellt;
R⁵³ und R⁵⁴ unabhängig voneinander ein Wasserstoffatom, einen Alkylrest, einen Aralkylrest darstellen, wobei die Arylgruppe ggf. durch einen oder mehrere gleiche oder verschiedene Substituenten substituiert ist, die ausgewählt sind aus: eine OH-Gruppe, Halogen, Nitro, Alkyl, Alkoxy, -NR⁵⁵R⁵⁶,
R⁵⁵ und R⁵⁶ unabhängig voneinander ein Wasserstoffatom, einen Alkylrest oder eine -COR⁵⁷-Gruppe darstellen oder R⁵⁵ und R⁵⁶ zusammen mit dem Stickstoffatom, an das sie gebunden sind, einen ggf. substituierten Heterocyclus bilden,
R⁵⁷ ein Wasserstoffatom, einen der Reste Alkyl, Alkoxy oder -NR⁵⁸R⁵⁹ darstellt,
R⁵⁸ und R⁵⁹ unabhängig voneinander ein Wasserstoffatom oder einen Alkylrest darstellen oder R⁵⁸ und R⁵⁹ zusammen mit dem Stickstoffatom, an das sie gebunden sind, einen ggf. substituierten Heterocyclus bilden;
wobei p eine ganze Zahl zwischen 0 und 6 ist;
Het den Rest Tetrahydrofuran-3-yl darstellt,
sowie die Additionssalze mit den mineralischen und organischen Säuren oder mit den mineralischen und organischen Basen dieser Verbindungen der allgemeinen Formel (I),
mit Ausnahme der Verbindungen der Formel (I), in denen, wenn Het Tetrahydrofuran oder Tetrahydropyran, R¹ den Rest OR³, wobei R³ ein Wasserstoffatom, einen der Reste Alkyl, Arylalkyl, Heterocycloalkylcarbonyl darstellt, wobei der Rest Heterocycloalkyl durch ein Kohlenstoffatom, Alkylcarbonyl, Arylcarbonyl oder Aralkylcarbonyl verzweigt ist, R² ein Wasserstoffatom und Y den Rest -(CH₂)ₚ-, wobei p = 0 ist, darstellt, dann stellt X -CO-N (R⁴⁵) -C (R⁴⁶R⁴⁷) -CO-, mit R⁴⁵=R⁴⁶=H, dar.

2. Verbindungen nach Anspruch 1, **dadurch gekennzeichnet, dass** R¹ ein Wasserstoffatom, den Rest -OR³ oder Oxo darstellt.

3. Verbindungen nach einem der vorhergehenden Ansprüche, **dadurch gekennzeichnet, dass** X -(CH₂)ₙ-, -(CH₂)ₙ-CO-, -O- (CH₂)ₙ-CO-, -CO-N(R⁴⁵) -D-CO-, -N(R⁴⁵)-CO-(CH₂)ₙ-CO-, -N (R⁴⁵)-CO-C(R⁴⁶R⁴⁷)-CO-, -N(R⁴⁵)-CO-NH-C (R⁴⁶R⁴⁷) -CO-, -N(R⁴⁵)-(CH₂)ₙ-CO-, -CO-N(R⁴⁵)-C(R⁴⁶R⁴⁷)-CO oder -Z-CO- darstellt.

4. Verbindungen nach Anspruch 3, **dadurch gekennzeichnet, dass** R⁴⁵ und R⁴⁷ ein Wasserstoffatom, R⁴⁶ ein Wasserstoffatom, einen Alkyl- oder Phenylrest, D einen Phenylenrest und Z einen Thiazolrest darstellen.

5. Verbindungen nach einem der vorhergehenden Ansprüche, **dadurch gekennzeichnet, dass** R² ein Wasserstoffatom oder einen Arylrest und vorzugsweise Benzyl darstellt.

6. Verbindungen nach einem der vorhergehenden Ansprüche, **dadurch gekennzeichnet, dass** A A1 darstellt, wobei W ein Schwefelatom darstellt.

7. Verbindungen nach einem der vorhergehenden Ansprüche, **dadurch gekennzeichnet, dass** A den Rest 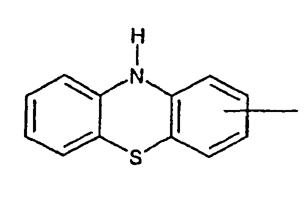
darstellt.

8. Verbindungen, die einer der folgenden Formeln entsprechen:
N-[(1S)-1-({[(3S)-2-Hydroxytetrahydro-3-furanyl]amino}-carbonyl)-3-methylbutyl]-10*H*-phenothiazin-2-carboxamid;
(3*S*)-3-({(2*S*)-4-Methyl-2-[(10*H*-phenothiazin-2-ylcarbonyl)-amino]pentanoyl}amino)tetrahydro-2-furanylacetat;
*N*-[(3S)-2-Hydroxytetrahydro-3-furanyl]-2-(10H-phenothiazin-2-yl)-1,3-thiazol-4-carboxamid;
*N*-[4-({[(3*S*)-2-Hydroxytetrahydro-3-furanyl]amino}carbonyl)-phenyl]-10*H*-phenothiazin-2-carboxamid;
N-[(1S)-1-({[(3S)-2-Hydroxytetrahydro-3-furanyl]amino}-carbonyl)-3-methylbutyl]-10*H*-phenothiazin-1-carboxamid;
(3S)-3-({(2S)-4-Methyl-2-[(10*H*-phenothiazin-2-ylcarbonyl)-amino]pentanoyl}amino)tetrahydro-2-furanylpivalat;
(3S)-3-({(2S)-4-Methyl-2-[(10*H*-phenothiazin-2-ylcarbonyl)-amino]pentanoyl}amino)tetrahydro-2-furanyl-3,3-dimethylbutanoat;
(3S)-3-({(2S)-4-Methyl-2-[(10*H*-phenothiazin-2-ylcarbonyl)-amino]pentanoyl}amino)tetrahydro-2-furanylbenzoat;
(3S)-3-({(2S)-4-Methyl-2-[(10*H*-phenothiazin-2-ylcarbonyl)-amino] pentanoyl} amino) tetrahydro-2-furanylphenylacetat ;
(3S) -3- ({(2S) -4- Methyl-2-[(10*H*-phenothiazin-2-ylcarbonyl)-amino] pentanoyl}amino)tetrahydro-2-furanyl(2*S*)-2-(dimethylamino)-3-phenylpropanoat;
(3*S*)-3-({(2*S*)-4-Methyl-2-[(10*H*-phenothiazin-2-ylcarbonyl)-amino] pentanoyl} amino) tetrahydro-2-furanyl-4-morpholincarboxylat;

9. Verbindungen, die einer der folgenden Formeln entsprechen:
N-[(1S)-1-({[(3S)-2-Hydroxytetrahydro-3-furanyl]amino}-carbonyl)-3-methylbutyl]-10*H*-phenothiazin-2-carboxamid;
(3S)-3-({(2S)-4-Methyl-2-[(10*H*-phenothiazin-2-ylcarbonyl)-amino]pentanoyl}amino)tetrahydro-2-furanyl-acetat;
(2R,3S)-3-({(2S)-4-Methyl-2-[(10*H*-phenothiazin-2-ylcarbonyl)-amino]pentanoyl}amino)tetrahydro-2-furanylacetat;
(2S,3S)-3-({(2S)-4-Methyl-2-[(10*H*-phenothiazin-2-ylcarbonyl)-amino]pentanoyl}amino)tetrahydro-2-furanylacetat; sowie die Additionssalze mit den mineralischen und organischen Säuren oder mit den mineralischen und organischen Basen dieser Verbindungen.

10. Verbindungen nach einem der Ansprüche 1 bis 9 in Form von Medikamenten.

11. Pharmazeutische Zusammensetzungen, die als Wirkstoff mindestens ein Medikament nach Anspruch 10 umfassen.

12. Verwendung von Verbindungen der Formel (Iₐ), wie sie im Nachstehenden definiert ist, 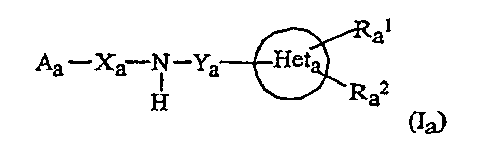
in Form von Racemat, Enantiomeren, Diastereoisomeren oder allen Kombinationen dieser Formen, in der
Rₐ¹ ein Wasserstoffatom, einen Rest -OR³, -SR³, Oxo oder ein cyclisches Acetal darstellt,
wobei R³ ein Wasserstoffatom, einen der Reste Alkyl, Arylalkyl, Heterocycloalkylcarbonyl, Alkylcarbonyl, Arylcarbonyl oder Aralkylcarbonyl darstellt,
wobei die Reste Alkyl, Aryl oder Heterocycloalkyl ggf. durch einen oder mehrere gleiche oder verschiedene Substituenten substituiert sind, die ausgewählt sind aus: Alkyl, OH, Alkoxy, Nitro, Cyano, Halogen oder -NR⁴R⁵ ;
R⁴ und R⁵ unabhängig voneinander ein Wasserstoffatom oder einen Alkylrest darstellen oder R⁴ und R⁵ zusammen mit dem Stickstoffatom, an das sie gebunden sind, einen ggf. substituierten Heterocyclus bilden,
Rₐ² ein Wasserstoffatom, einen der Reste Alkyl, Aryl oder Aralkyl darstellt, wobei die Arylgruppe ggf. durch einen oder mehrere gleiche oder verschiedene Reste substituiert ist, die ausgewählt sind aus: -OR⁶, -NR⁷R⁸, Halogen, Cyano, Nitro oder Alkyl,
wobei R⁶, R⁷ und R⁸ unabhängig voneinander ein Wasserstoffatom, einen der Reste Alkyl, Aryl, Aralkyl, Alkylcarbonyl, Arylcarbonyl oder Aralkylcarbonyl darstellen;
Aₐ einen Rest A1 darstellt 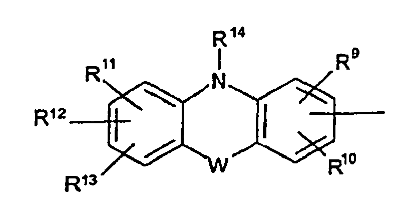
bei dem R⁹, R¹⁰, R¹¹, R¹², R¹³ unabhängig voneinander ein Wasserstoffatom, ein Halogen, die OH-Gruppe, einen der Reste Alkyl, Alkoxy, Cyano, Nitro oder -NR¹⁵R¹⁶ darstellen,
R¹⁵ und R¹⁶ unabhängig voneinander ein Wasserstoffatom, einen Alkylrest oder eine -COR¹⁷-Gruppe darstellen oder R¹⁵ und R¹⁷ zusammen mit dem Stickstoffatom, an das sie gebunden sind, einen ggf. substituierten Heterocyclus bilden,
R¹⁷ ein Wasserstoffatom, einen der Reste Alkyl, Alkoxy oder -NR¹⁸R¹⁹ darstellt,
R¹⁸ und R¹⁹ unabhängig voneinander ein Wasserstoffatom oder einen Alkylrest darstellen oder R¹⁸ und R¹⁹ zusammen mit dem Stickstoffatom, an das sie gebunden sind, einen ggf. substituierten Heterocyclus bilden,
R¹⁴ ein Wasserstoffatom, einen Alkylrest oder eine -COR²⁰-Gruppe darstellt,
R²⁰ ein Wasserstoffatom, einen der Reste Alkyl, Alkoxy, Aryl, Aralkyl, Heterocycloalkyl oder -NR²¹R²² darstellt,
wobei die Reste Alkyl, Aryl oder Heterocycloalkyl ggf. durch einen oder mehrere gleiche oder verschiedene Substituenten substituiert sind, die ausgewählt sind aus: Alkyl, OH, Alkoxy, Nitro, Cyano, Halogen oder -NR⁴R⁵;
R²¹ und R²² unabhängig voneinander ein Wasserstoffatom oder einen Alkylrest darstellen oder R²¹ und R²² zusammen mit dem Stickstoffatom, an das sie gebunden sind, einen ggf. substituierten Heterocyclus bilden,
W eine Bindung, O oder S oder einen Rest -NR²³ darstellt, in dem R²³ ein Wasserstoffatom oder einen Alkylrest darstellt;
Xₐ-(CH₂)ₙ-, -(CH₂) ₙ-CO-, -N(R⁴⁵)-CO-(CH₂)ₙ-CO-, -N(R⁴⁵)-CO-D-CO-, -CO-N(R⁴⁵)-D-CO-, -CO-D-CO-, -CH=CH-(CH₂)ₙ-CO-, -N(R⁴⁵)-(CH₂)ₙ-CO-, -N(R⁴⁵)-CO-C(R⁴⁶R⁴⁷)-CO-, -O-(CH₂)ₙ-CO-, -N(R⁴⁵)-CO-NH-C(R⁴⁶R⁴⁷)-CO-, -CO-N(R⁴⁵)-C(R⁴⁶R⁴⁷)-CO-, -S-(CH₂) ₙ-CO- oder -Z-CO- darstellt;
D einen Phenylenrest darstellt, der ggf. durch einen oder mehrere gleiche oder verschiedene Reste substituiert ist, die aus Alkyl, Alkoxy, OH, Nitro, Halogen, Cyano oder ggf. durch einen Alkylrest verestertem Carboxyl ausgewählt sind;
Z einen Heterocyclus darsellt,
R⁴⁵ ein Wasserstoffatom oder einen Alkylrest darstellt,
R⁴⁶ und R⁴⁷ unabhängig voneinander ein Wasserstoffatom, einen der Reste Alkyl, Aryl oder Aralkyl darstellen, wobei die Gruppen Alkyl und Aryl ggf. durch einen oder mehrere gleiche oder verschiedene Substituenten substituiert sind, die ausgewählt sind aus: einer OH-Gruppe, -SH, Halogen, Nitro, Alkyl, Alkoxy, Alkylthio, Aralkoxy, Arylalkylthio, -NR⁴⁸R⁴⁹ und ggf. durch einen Alkylrest verestertes Carboxyl ;
R⁴⁸ und R⁴⁹ unabhängig voneinander ein Wasserstoffatom, einen Alkylrest oder eine -COR⁵⁰-Gruppe darstellen oder R⁴⁸ und R⁴⁹ zusammen mit dem Stickstoffatom, an das sie gebunden sind, einen ggf. substituierten Heterocyclus bilden,
R⁵⁰ ein Wasserstoffatom, einen der Reste Alkyl, Alkoxy oder -NR⁵¹R⁵² darstellt,
R⁵¹ und R⁵² unabhängig voneinander ein Wasserstoffatom oder einen Alkylrest darstellen oder R⁵¹ und R⁵² zusammen mit dem Stickstoffatom, an das sie gebunden sind, einen ggf. substituierten Heterocyclus bilden;
wobei n eine ganze Zahl zwischen 0 und 6 ist;
Yₐ-(CH₂)ₚ-, -C(R⁵³R⁵⁴)-(CH₂)ₚ-, -C(R⁵³R⁵⁴)-CO- darstellt;
R⁵³ und R⁵⁴ unabhängig voneinander ein Wasserstoffatom, einen Alkylrest, einen Aralkylrest darstellt, wobei die Arylgruppe ggf. durch einen oder mehrere gleiche oder verschiedene Substituenten substituiert ist, die ausgewählt sind aus: eine OH-Gruppe, Halogen, Nitro, Alkyl, Alkoxy, -NR⁵⁵R⁵⁶,
R⁵⁵ und R⁵⁶ unabhängig voneinander ein Wasserstoffatom, einen Alkylrest oder eine -COR⁵⁷-Gruppe darstellen oder R⁵⁵ und R⁵⁶ zusammen mit dem Stickstoffatom, an das sie gebunden sind, einen ggf. substituierten Heterocyclus bilden,
R⁵⁷ ein Wasserstoffatom, einen der Reste Alkyl, Alkoxy oder -NR⁵⁸R⁵⁹ darstellt,
R⁵⁸ und R⁵⁹ unabhängig voneinander ein Wasserstoffatom oder einen Alkylrest darstellen oder R⁵⁸ und R⁵⁹ zusammen mit dem Stickstoffatom, an das sie gebunden sind, einen ggf. substituierten Heterocyclus bilden;
wobei p eine ganze Zahl zwischen 0 und 6 ist;
Hetₐ den Rest Tetrahydrofuran-3-yl darstellt,
sowie die Additionssalze mit den mineralischen und organischen Säuren oder mit den mineralischen und organischen Basen dieser Verbindungen der allgemeinen Formel (I),
für die Herstellung von Medikamenten zur Behandlung von Krankheiten, an denen die Calpaine und/oder die reaktiven Formen des Sauerstoffs beteiligt sind.

13. Verwendung von Verbindungen der Formel (Iₐ) nach Anspruch 12 für die Herstellung von Medikamenten für die Behandlung von Krankheiten, an denen die reaktiven Formen des Sauerstoffs beteiligt sind.

14. Verwendung von Verbindungen der Formel (Iₐ) nach Anspruch 12 für die Herstellung von Medikamenten für die Behandlung von Krankheiten, an denen die reaktiven Formen des Sauerstoffs und die Calpaine beteiligt sind.

15. Verwendung von Verbindungen der Formel (Iₐ) nach einem der Ansprüche 12 bis 14 für die Herstellung von Medikamenten für die Behandlung von entzündlichen und immunologischen Krankheiten, kardiovaskulären und zerebrovaskulären Krankheiten, Störungen des zentralen oder peripheren Nervensystems, der Osteoporose, von Muskeldystrophien, proliferativen Krankheiten, des grauen Stars, von Organtransplantationen, Autoimmun- und Viruserkrankungen, Krebs und allen Krankheiten, die durch eine Überproduktion der ROS und/oder eine Aktivierung der Calpaine **gekennzeichnet** sind.

16. Verwendung von Verbindungen der Formel (Iₐ) nach einem der Ansprüche 12 bis 15, **dadurch gekennzeichnet, dass** R¹ ein Wasserstoffatom, den Rest -OR³ oder Oxo darstellt.

17. Verwendung von Verbindungen der Formel (Iₐ) nach einem der Ansprüche 12 bis 16, **dadurch gekennzeichnet, dass** X -(CH₂)ₙ-, -(CH₂)ₙ-CO-, -O-(CH₂)ₙ-CO-, -CO-N(R⁴⁵)-D-CO-, -N(R⁴⁵)-CO-(CH₂)ₙ-CO-, -N(R⁴⁵)-CO-C(R⁴⁶R⁴⁷)-CO-, -N(R⁴⁵)-CO-NH-C(R⁴⁶R⁴⁷)-CO-, -N(R⁴⁵)-(CH₂)ₙ-CO-, -CO-N(R⁴⁵)-C(R⁴⁶R⁴⁷)-CO oder -Z-CO- darstellt.

18. Verwendung von Verbindungen der Formel (Iₐ) nach Anspruch 17, **dadurch gekennzeichnet, dass** R⁴⁵ und R⁴⁷ ein Wasserstoffatom, R⁴⁶ ein Wasserstoffatom, einen Alkyl- oder Phenylrest, D einen Phenylenrest und Z einen Thiazolrest darstellen.

19. Verwendung von Verbindungen der Formel (Iₐ) nach einem der Ansprüche 12 bis 18, **dadurch gekennzeichnet, dass** R² ein Wasserstoffatom oder einen Aralkylrest und vorzugsweise Benzyl darstellt.

20. Verwendung von Verbindungen der Formel (Iₐ) nach einem der Ansprüche 12 bis 19, **dadurch gekennzeichnet, dass** A A1 darstellt, wobei W ein Schwefelatom darstellt.

21. Verwendung von Verbindungen der Formel (Iₐ) nach einem der Ansprüche 12 bis 20, **dadurch gekennzeichnet, dass** A den Rest 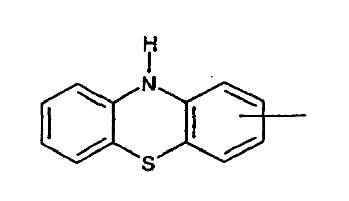
darstellt.

22. Verwendung von Verbindungen der Formel (Iₐ) nach einem der Ansprüche 12 bis 21, **dadurch gekennzeichnet, dass** sie einer der folgenden Formeln entsprechen:
N-[(1S)-1-({[(3S)-2-Hydroxytetrahydro-3-furanyl]amino}-carbonyl)-3-methylbutyl]-10*H*-phenothiazin-2-carboxamid;
(3S)-3-({(2S)-4-Methyl-2-[(10*H*-phenothiazin-2-ylcarbonyl)-amino]pentanoyl}amino)tetrahydro-2-furanylacetat;
N-[(3S)-2-Hydroxytetrahydro-3-furanyl]-2-(10H-phenothiazin-2-yl)-1,3-thiazol-4-carboxamid;
N-[4-({[(3S)-2-Hydroxytetrahydro-3-furanyl]amino}carbonyl)-phenyl]-10*H*-phenothiazin-2-carboxamid;
N-[(1S)-1-({[(3S)-2-Hydroxytetrahydro-3-furanyl]amino}-carbonyl)-3-methylbutyl]-10*H*-phenothiazin-1-carboxamid;
(3S)-3-({(2S)-4-Methyl-2-[(10*H*-phenothiazin-2-ylcarbonyl)-amino]pentanoyl}amino)tetrahydro-2-furanylpivalat;
(3S)-3-({(2S)-4-Methyl-2-[(10*H*-phenothiazin-2-ylcarbonyl)-amino]pentanoyl}amino)tetrahydro-2-furanyl-3,3-dimethylbutanoat;
(3S)-3-({(2*S*)-4-Methyl-2-[(10*H*-phenothiazin-2-ylcarbonyl)-amino]pentanoyl}amino)tetrahydro-2-furanylbenzoat;
(3S)-3-({(2S)-4-Methyl-2-[(10*H*-phenothiazin-2-ylcarbonyl)-amino]pentanoyl}amino)tetrahydro-2-furanylphenylacetat;
(3S)-3-({(2S)-4-Methyl-2-[(10*H*-phenothiazin-2-ylcarbonyl)-amino] pentanoyl}amino) tetrahydro-2-furanyl (2S)-2-(dimethylamino)-3-phenylpropanoat;
(3S)-3-({(2S)-4-Methyl-2-[(10*H*-phenothiazin-2-ylcarbonyl)-amino]pentanoyl}amino)tetrahydro-2-furanyl-4-morpholincarboxylat;

23. Verwendung von Verbindungen der Formel (Iₐ) nach einem der Ansprüche 12 bis 22, **dadurch gekennzeichnet, dass** sie einer der folgenden Formeln entsprechen:
N-[(1S)-1-({[(3S)-2-Hydroxytetrahydro-3-furanyl]amino}-carbonyl)-3-methylbutyl]-10*H*-phenothiazin-2-carboxamid;
(3S)-3-({(2S)-4-Methyl-2-[(10*H*-phenothiazin-2-ylcarbonyl)-amino]pentanoyl}amino)tetrahydro-2-furanylacetat;
(2R,3S)-3-({(2S)-4-Methyl-2-[(10*H*-phenothiazin-2-ylcarbonyl)amino]pentanoyl}amino)tetrahydro-2-furanylacetat;
(2S,3S)-3-({(2S)-4-Methyl-2-[(10*H*-phenothiazin-2-ylcarbonyl)-amino]pentanoyl}amino)tetrahydro-2-furanylacetat.

24. Die Verbindungen, die einer der folgenden Formeln entsprechen, in Form von neuen Industrieprodukten:
N-1-(4-Anilinophenyl)-N-4-[(3S)-2-oxotetrahydro-3-furanyl]succinamid;
(2S)-2-{[4-(4-Anilinoanilino)-4-oxobutanoyl]amino}-4-methylmethylpentanoat;
N-1-(4-Anilinophenyl)-N4-[(1S)-1-formyl-3-methylbutyl]-succinamid;
Benzyl-3-(4-Anilinoanilino)-3-oxo-2-phenylpropanoat;
3-(4-Anilinoanilino)-3-oxo-2-phenylpropansäure;
N-1-(4-Anilinophenyl)-N-3-[(3S)-2-oxotetrahydro-3-furanyl]-2-phenylmalonamid;
3- (4-Anilinoanilino)dihydro-2(3H)-furanon;
(2S)-4-Methyl-2-[(10*H*-phenothiazin-2-ylcarbonyl)amino]-methylpentanoat;
(2S)-4-Methyl-2-[(10*H*)-phenothiazin-2-ylcarbonyl)amino]-pentansäure;
N-[(1*S*)-3-Methyl-1-({[(3*S*)-2-oxotetrahydro-3-furanyl]-amino} carbonyl) butyl]-10*H*-phenothiazin-2-carboxamid ;
2-(10*H*-Phenothiazin-2-yl)-1,3-thiazol-4-ethylcarboxylat ;
2-(10*H*-Phenothiazin-2-yl)-1,3-thiazol-4-carbonsäure;
N-[(3S)-2-Oxotetrahydro-3-furanyl]-2-(10*H*-phenothiazin-2-yl)-1,3-thiazol-4-carboxamid;
4-[(10*H*-Phenothiazin-2-ylcarbonyl)amino]methylbenzoat;
4-[(10H-Phenothiazin-2-ylcarbonyl}amino]benzoesäure;
N-[4-({[(3*S*)-2-Oxotetrahydro-3-furanyl]amino}carbonyl)-phenyl]-10*H*-phenothiazin-2-carboxamid;
(2S)-4-Methyl-2-[(10*H*-phenothiazin-1-ylcarbonyl)amino]-methylpentanoat;
(2S)-4-Methyl-2-[(10*H*-phenothiazin-1-ylcarbonyl)amino]-pentansäure;
N-[(1S)-1-({[(3S)-2-Oxotetrahydro-3-furanyl]amino}-carbonyl)-3-methylbutyl]-10*H*-phenothiazin-1-carboxamid;
*N*-[(1*S*)-1-(1,4-Dioxa-7-azaspiro[4,4]non-7-ylcarbonyl)3-methylbutyl]-10*H*-phenothiazin-2-carboxamid;
2-(3,5-di-*tert*-Butyl-4-hydroxyphenoxy)-N-[(3S)-2-oxotetrahydro-3-furanyl]acetamid;
5-Nitro-1-propylindolin;
1-Propyl-2,3-dihydro-1*H*-indol-5-ylamin;
3-Oxo-2-phenyl-N-(1-propyl-2,3-dihydro-1*H*-indol-5-yl)-betaalanin;
N¹-[(3S)-2-Oxotetrahydro-3-furanyl]-2-phenyl-N³-(1-propyl-2,3-dihydro-1*H*-indol-5-yl)malonamid;
N-(2-Anilinophenyl)-N'-[(3S)-2-oxotetrahydro-3-furanyl]-harnstoff;
Oxo[(1-propyl-2,3-dihydro-1*H*-indol-5-yl)amino] ethylacetat ;
Oxo[(1-propyl-2,3-dihydro-1*H*-indol-5-yl)amino]essigsäure ;
(2S)-2-({[(2R)-6-Hydroxy-2,5,7,8-tetramethyl-3,4-dihydro-2*H*-chromen-2-yl]carbonyl}amino)-3-phenylmethylpropanoat ;
(2R)-N-[(1S)-1-Benzyl-2-oxoethyl]-6-hydroxy-2,5,7,8-tetramethyl-3,4-dihydro-2*H*-chromen-2-carboxamid ;
5-Methyl-1,5-indolin-*tert*-butylcarboxylat;
1-(*tert*-Butoxycarbonyl)-5-indolincarbonsäure;
5-({[(3S)-2-Oxotetrahydro-3-furanyl]amino}carbonyl)-1-indolin-*tert*-butylcarboxylat ;
5-({[(3*S*)-2-Hydroxytetrahydro-3-furanyl]amino}carbonyl)-1-indolin-*tert*-butylcarboxylat.
